# EUROPEAN PATENT APPLICATION

(11) **EP 1 101 457 A2**
(43) Date of publication of application: **23.05.2001**
(21) Application number: 01200633.4
(22) Date of filing: 16.09.1992
(51) Int. Cl.: A61F 2/06

(54) **Controlled porosity implantable primary lumen device**

(30) Priority: 16.09.1991 US 760716; 16.09.1991 US 760717; 16.09.1991 US 760718; 16.09.1991 US 760728; 16.09.1991 US 760753
(62) Divisional of application: 92920322.2
(71) Applicant: Atrium Medical Corporation, Hudson, NH 03051 (US)
(72) Inventor: Herweck, Steve A., Nashua, NH 03062 (US); Karwoski, Theodore, Hollis, NH 03049 (US); Martakos, Paul, Pelham, NH 03076 (US)
(74) Representative: Greenwood, John David

(57) **Abstract**

An implantable prosthetic device for connection to a fluid flow pathway of a patient, the device comprising an implantable body made of a single biocompatible material and extruded as a single body with microporous interior walls, said extruded single body having a shape to attach to said fluid flow pathway, said single body further having a multiplicity of capillary lumina (314) therein defined by said microporous interior walls (315) and extending substantially parallel to each other along a longitudinal axis of the body for accommodating fluid flow along said axis when attached to said fluid flow pathway.

## Description

### Background of the Invention

The present invention relates to implantable prostheses, and particularly to implantable prostheses having a passageway, or lumen for carrying a biological fluid. In its simplest form the invention may relate to a vessel and the biological fluid may be blood, but in other embodiments, the prosthesis may constitute a patch or graft of an organ, with the lumen defining an organ-related fluid path carrying some other biological fluid, such as an enzyme, hormone, secretion or waste product made or processed by the organ. In the prior art, a great deal of research and experimentation have been performed with vascular grafts to identify materials which have the necessary structural properties yet are compatible with cellular growth such that they may immediately function as blood vessels, yet provide a structural framework on which vessel tissue may regenerate. In various embodiments of the invention, as described more fully below, applicant describes a unique structure capable of supporting tissue growth, yet having a functional geometry including a lumen.

One type of implantable device is a synthetic vascular graft such as is commonly used to replace damaged or dysfunctional arterial or venous pathways, for example at the site of an aneurysm or occlusion. Bypass grafts are often used to divert blood flow around damaged regions to restore blood flow. Another use of vascular prostheses is for creating a bypass shunt between an artery and vein, specifically for multiple needle access, such as is required for hemodialysis treatments. Following multiple percutaneous invasions into a vein, the vein may either collapse along the puncture track or become aneurysmal, leaky or fill with clot, causing significant risk of pulmonary embolization. Vascular prostheses have been used for many years as an alternative to patients' own veins for vascular access during hemodialysis.

Materials research has led to the development of some synthetic materials for use in artificial vascular prostheses. For example, polytetrafluoroethylene (PTFE), a polymeric material which may be stretched to a specific length and expanded to a specific thickness, is often used to fabricate single lumen artificial veins and arteries. When thus stretched, or expanded, PTFE forms a network of interrelated nodes and fibrils. The diameters of the fibrils and internodal distances vary depending upon the conditions and rate at which the PTFE is stretched and/or expanded. Typical stretched and/or expanded PTFE articles have an internodal distance ranging from approximately twenty to approximately thirty microns.

An advantage of stretched and/or expanded PTFE is that the diameters of the fibrils can be made much smaller than the diameters of fibrils of knitted or woven fabrics which have previously been used for vascular prostheses. Moreover, due to the ability to control the pore diameter and porosity of PTFE tubing used, for example, for vascular prostheses, it is possible to decrease the occurrence of thrombosis associated therewith. Typically, however, PTFE vascular grafts cannot safely be used to withdraw blood until they have been in place in the body for a minimum of 14 days after surgery and have become surrounded by fibrotic tissue. This is because bleeding occurs at the site of a needle puncture in PTFE grafts if fibrotic tissue is absent. Complications which can result from early puncturing of PTFE arteriovenous fistulas include a hematoma surrounding the graft, false aneurysm, and graft occlusion.

Various other synthetic materials, in addition to PTFE, have been used for vascular grafts, including Dacron® brand and other synthetic polyester fibers, mandrel spun polyurethane, and silicon elastomer fibers. Additionally, vascular grafts have been formed using autologous saphenous vein, modified bovine carotid xenograft, and modified human umbilical vein. None, however, has overcome the problems associated with early failure of the graft following implantation.

In an effort to address these problems, various types of vascular access devices have been developed. One example of such a device is described by Tesio in U.S. Patent No. 4,898,669 (Feb. 6, 1990). This device is a catheter system mechanically coupled with a prosthetic vascular graft for use in blood purification. Devices available for long-term, repeated vascular access allow a catheter to be introduced to a blood flow pathway, but they do not generally allow chronic, permanent implantation. In some cases, due to the materials used, an autoimmune response occurs resulting in the formation of an occlusive hematoma.

Another vascular graft is disclosed in U.S. Patent No. 4,619,641 (Oct. 28, 1986) to Shanzer which discloses a coaxial double lumen device for use in hemoaccess. The space between the two lumena is filled with a self-sealing, non-biodegradable polymer which does not permit escaped bleeding following needle puncture. The Shanzer product consists of an outer tube positioned over an inner tube, both tubes being made of expanded PTFE.

While research has also led to the development of some improved drug delivery systems, these systems still require a significant amount of medication to achieve a therapeutic level for an organ specific function such as is required for a whole body systemic effect. Conventional drug delivery systems, for example, including orally applied tablets and liquids, injections, and localized intravenous infusions, all must be applied in rather large doses to achieve a systemic effect. Introduction of a catheter directly into a blood flow pathway, for example, results in delivery of bolus amounts of drug to a patient's system. This results in uncontrolled and often varied, whole body physiological effects, as well as blood vessel wall injury at the site of penetration. Much of the current research in drug delivery, therefore, has been directed to allowing delivery of drugs to a patient in a controlled, non-bolus manner, and in generally smaller doses. This requires that delivery be localized, or targeted, to a cell specific organ.

Presently, material research has also enabled modern developments such as transdermal and time-release delivery systems which also create a whole body systemic effect. Future research suggests the extended use of chemically modified drugs complexed to carrier or bioactive agents, organic vesicles, and controlled delivery systems such as micro-pumps and mini-pumps. These and other new drug delivery systems are discussed, for example, by R. Langer, In: "New Methods of Drug Delivery", Science 249:1527-1533 (September 28, 1990).

Additionally, recently published evidence indicating that cellular activities are controlled by receptors, "molecular switches" on the membrane surface of cells suggests that bioactive and pharmaceutical drug interactions, whether initiators or inhibitors, can be utilized to improve implantable organ and autogenous organ transplant performance. These receptors control cellular activities by binding with highly specific substances referred to as "ligands." Like the action of a key in a lock, ligands fit into receptors and, if the fit is precise, turn on or off certain cellular processes. Some ligands act as antagonists to inhibit cellular activities by blocking a receptor. Research has shown that many aspects of cardiovascular disease are controlled by specific cell surface receptors.

Another problem associated with known vascular grafts stems from their delicate structure making them difficult to handle and position properly during surgery. Due to their circumferentially uniform appearance, the grafts may be twisted during implantation, which can reduce the openness, or patency, of the implanted graft.

Another problem associated with known vascular grafts is that they are transparent to known non-surgical techniques for viewing that are generally used to detect structures in the body. These techniques include x-rays, MRI scanning, fluoroscopy, ultrasound, and nuclear magnetic resonance. As a result, post-implantation examination of known vascular grafts is difficult. If a surgeon suspects that a blockage of an implanted vascular graft has occurred, for example, she must inject radiopaque dye into the patient through the graft. The dye allows the graft to be visible during fluoroscopic examination to determine whether it has collapsed or is satisfactorily transporting blood flow. This process is invasive to the patient and places further burdens on the patient's circulatory system.

Various marking devices such as metal tabs which are sutured to a prosthesis, have been developed to avoid the need for injecting a radioopaque dye into a patient's bloodstream. However, there remains a need for an improved system of indicating the patency of a graft vessel.

Other problems associated with vascular grafts formed of known materials and configurations are that their biocompatibility, non-thrombogenic potential, cell harboring, and seeding properties are limited. Specifically, for example, intimal hyperplasia, which is a naturally occurring phenomenon characterized by progressive cellular closure of a blood vessel lumen, threatens the patency of almost all known vascular graft material. Even when surgically repaired or exposed to less intensive manipulative techniques such as balloon dilation, mechanical dilation, laser ablation or mechanical dissection by anthrectomy, intimal hyperplasia is the primary cause of stenosis of all implantable vascular grafts and restenosis of natural arteries following repair of diseased blood vessels.

One cause of intimal hyperplasia is the proliferation of smooth muscle cells into the lumen of the graft. Other causes include injury to the venous system and/or arterial circulation network, caused by trauma, disease, or systemic factors such as hypercholesterolemia.

While intimal hyperplasia is known to cause significant lumenal obstruction of vascular grafts, the detailed cellular mechanisms leading to smooth muscle cell proliferation are not completely understood. It is believed, however, that growth factors such as platelet derived growth factor (PDGF) initiate a number of intracellular events, called "regulatory signals." These signals include the activation of protein kinase C. Additionally, different growth-stimulating factors are thought to initiate different signals. These different signals lead to a set of common pathways which stimulate DNA synthesis. Such pathways are called obligatory events." It is not clear which of these obligatory events, though, are responsible for intimal hyperplasia.

Also, lumenal blood vessel injury, whether micro-capillary (0.5 mm or less diameter) or aortic (up to 30 mm diameter), induced by trauma, such as mechanical stress, or progressive disease states such as arteriosclerosis or mechanical hemodynamic stress, causes activation of platelets, injury and necrosis of smooth muscle and endothelial cells, and resultant leukocyte infiltration. These events result in the production and release of factors that stimulate smooth muscle cell migration and proliferation from adjacent tissue, subsequently leading to intimal hyperplasia. As stated, such growth, when induced by trauma or progressive disease, without pharmacologic intervention, causes stenotic closure and failure of most autogenous organ transplantations such as coronary artery bypass grafting and synthetic implantable vascular graft devices.

Known treatments of intimal hyperplasia involve the administration of various drugs that inhibit muscle cell proliferation. For example, somatostatin inhibits tumor cell growth. Angiopeptin, a synthetic peptide analog of somatostatin, reduces myointimal proliferation. Trapidil, an antianginal agent possessing vasodilatory and antiplatelet properties, and Terbinafine, an antifungal agent, both are effective antiproliferative agents. Colchicine, a drug which possesses antimitotic and antisecretory properties, also is effective in reducing myointimal thickening.

Besides antiproliferative agents, drugs that inhibit the synthesis and secretion of extracellular matrix are also useful, since a large proportion of the restenotic tissue is composed of extracellular matrix. Because smooth muscle cell migration is an essential step in intimal proliferation, agents that inhibit SMC migration ultimately inhibit proliferation.

Other techniques have been developed, for improving the patency of implantable devices. One such process is glow discharge polymerization as taught by United States Patent 4,632,842 to Karwoski et al. Karwoski teaches coating, by the use of glow discharge conducted in a tubular reaction vessel, the surface of an elongate organic substrate with substantially uniform, very low surface-energy coating. Still, even with the Karwoski teaching, the proliferation of smooth muscle cells into known, prosthetic vascular grafts is not controlled or reduced sufficiently to prevent intimal hyperplasia

Another problem associated with known prosthetic devices arises in connection with the use of such devices as a means for drug delivery. That is, while it is known that various prostheses can be coated with bioactive and pharmaceutic agents for blood contact, the limited blood contact surface area within the single lumen of known prostheses used for this purpose limits the amount of agent that can be effectively distributed into the flow through body fluid. Additionally, the high ratio of blood flow-through to contact surface area results in a high level of wash-off.

In addition to the foregoing limitations of the prior art, certain inconveniences or shortcomings bear further note.

During prosthetic surgery, for example, in the course of replacing or by-passing damaged arteries and veins, the need often arises to have prosthetic devices of different diameters. In the physical setting, arteries are generally smaller in diameter than veins, thus requiring arterial and venous grafts to be available in a range of diameters. One way to provide grafts of different diameters to the surgeon at the time of implantation is to provide an array of individual vascular grafts each having a different diameter to the surgeon. These grafts are of a fine material, which can become entangled or torn during handling. In order to make these individual grafts communicate with each other, they must be sutured together.

According to another aspect of the invention it is therefore an object to-provide a vascular graft including at least two primary lumen-defining structures which can be manually separated from one another.

It is another object of the invention to provide such a graft wherein the lumena defined by the structures are of unequal diameters.

### Summary of the Invention

The invention features an implantable biocompatible device formed of a material of controlled porosity and having a primary lumen extending therethrough, and at least one secondary lumen, which as discussed further below, may according to different aspects be filled with materials, or may itself constitute another primary lumen.

In one aspect, the invention features an implantable, biocompatible prosthetic device for the sustained release of a drug or other bioactive material directly into a blood or other body fluid flow path. The device is a polymeric bi- or multilumenal tubular article which can be attached, for example, to an artery or vein to form a vascular graft or shunt. The device can also be used to provide organ to organ fluid communication. The device contains a primary lumen, which is dedicated to the flow of blood or other body fluid and at least one secondary lumen. The lumena are separated by a microporous, semi-permeable wall which permits passage of an agent from the secondary lumen to the primary lumen. Additionally, the microporosity of the separating wall promotes the growth, motility, and/or migration of cells into and through the wall.

The secondary lumen is particularly well suited to contain a material, such as a drug or other bioactive agent, which permeates the porous wall between the secondary lumen and the primary lumen over a sustained period of time. The rate at which the drug or other agent penetrates the porous wall is determined by several factors, including the size and number of the pores and the size of the drug molecule.

In one embodiment of this aspect of the invention, the prosthetic device comprises a tube which is adapted for attachment to a blood flow pathway, and for conducting the flow of blood therethrough. The tube has a biocompatible or bioinert exterior surface, and defines a primary lumen axially extending along the length of the tube, and at least one additional or secondary lumen. The secondary lumen is separated from the-first lumen by a microporous wall which allows a drug introduced into the secondary lumen to diffuse across the wall and into the primary lumen, and thus directly into the blood flow pathway. This is caused by patient cells penetrating the exterior wall of the tube and displacing air contained in the micropores of the tube's microporous structure. The displaced air, in turn, displaces material contained in the secondary lumen forcing the material to diffuse into the primary lumen.

- The secondary lumena can be pre-filled with a selected drug which time-diffuses or otherwise perfuses across the membrane. The lumena can also be filled with drug-producing cells which disperse a drug product into the secondary lumena, from which it then diffuses, as discussed above, into the primary lumen. The primary lumen and/or the secondary lumena can also be seeded with cells, for example endothelial cells, which proliferate at the site of attachment due to the cell to cell contact afforded through the walls of the microporous structure. The invention allows the endothelial cells to thrive, therefore, which helps to prevent undesired occlusion of the graft.

In another embodiment of this aspect of the invention, the tube features an external drug delivery device attached thereto by a second tube or catheter. The catheter is connected to the secondary lumen. The delivery device injects the drug into the secondary lumen from an external source. The device can be any of a variety of commercially and technologically available systems, such_as, for example, a biologically activated mini-pump which is either subcutaneously or extracutaneously located, or an external mechanical pump.

The present prosthetic device is preferably made from stretched and expanded polytetrafluoroethylene (PTFE). Stretched and expanded PTFE contains a porous network of nodes and fibrils which are created during the stretching and expansion process of porous tubing from PTFE. This porous network provides a semi-permeable wall or membrane between the lumena of the device.

In another aspect, the invention features a method for delivering directly into a fluid flow pathway in a controlled manner a bioactive material, such as a prophylactic or therapeutic drug, or a diagnostic material, such as a radiolabeled antibody. One embodiment of this aspect of the invention includes the steps of pre-filling one of the secondary lumena of the above-described implantable device with a bioactive material and implanting the device in a fluid flow pathway such as a vein or artery. In this manner, fluid flow is established through the primary lumen of the device. The pre-filled material diffuses across the wall or membrane separating the secondary lumena from the primary lumen in a controlled manner, thereby delivering the drug directly into the fluid, blood or otherwise, flowing through the primary lumen. The lumena may be prefilled by inserting a solid wire or rod of bioactive material releasably bound in a resorbable binder material.

In another embodiment of this aspect of the invention, a separate external drug delivery system is attached to at least one of the secondary lumena. This allows transport of the drug into the secondary lumen from an external source. The drug then diffuses across the interlumenal wall as described above.

The invention has several advantages. It allows implantable vascular grafting and controlled and/or continuous drug delivery to be combined. Additionally, the invention allows a bioactive substance to be delivered directly into a patient's bloodstream at a controlled rate without using intravenous injection, which generally must be performed in a hospital or doctor's office. The device and method allow a bioactive substance to be injected into the secondary lumen all at once from an external source, then released into the patient's bloodstream at a slower, continuous rate as the substance passes from the secondary lumen into the primary lumen of the graft. This sustained release of a substance into the bloodstream over time is less likely than known drug delivery methods to result in distal embolization. The graft provides a site for repeated cannulation which does not require directly accessing the bloodstream and therefore reduces the incidence of bleeding at the injection site.

In accordance with yet another aspect of the invention, the present invention features a self-sealing implantable prosthetic device for connection to a fluid flow pathway of a patient. The device comprises a tubular body adapted for attachment to the fluid flow pathway. The body defines a primary lumen for accommodating fluid flow therethrough and a secondary lumen partially circumscribing the primary lumen. The lumena share a common side wall. A non-biodegradeable elastomeric material is disposed in the secondary lumen. This permits repeated self-sealing penetrations by a cannula through the secondary lumen, the common side wall, and into the primary lumen.

It is a significant feature of this aspect of the invention, that the secondary lumen partially but not totally circumscribes the primary lumen. This provides a single piece unitary construction prosthetic device.

In a preferred embodiment, the present prosthetic device is made from stretched and/or expanded polytetraflouroethylene (PTFE). Stretched and/or expanded PTFE is made up of a network of nodes and fibrils which endow the PTFE with porosity. The network of nodes and fibrils created during the manufacture of tubing from PTFE results in a semi-permeable membrane having a permeability determined by the porosity of the PTFE material.

A method for repeatedly accessing a patient's vascular system is also the subject of this aspect of the present invention. The method includes the steps of implanting in the patient a structure constructed in accordance with the invention and accessing the patient's vascular system by passing a cannula through the second lumen, the common side wall, and into the first lumen of the structure.

In accordance with yet another aspect of the present invention an implantable prosthetic device, such as a vascular graft, has integral diagnostic indicia for determining the position and/or the patency or openness of the graft. The device comprises a biocompatible tubular body defining at least two lumena. A primary lumen is designated for blood flow, and at least one secondary lumen has a remotely detectible material, such as a radiopaque component or material different in density from the body, which is disposed within it. All of the lumena extend along the article's longitudinal axis. The remotely detectible component allows the position of the graft and/or the openness of the graft to be determined by non-invasive means. For example, fluoroscopy can be utilized in the case of a radiopaque material being disposed in a secondary lumen. On the other hand, MRI or ultrasonic examination can detect a material different in density from the body.

The detectible component can be, for example, a radiopaque material, such as a barium compound, which is injected into one of the secondary lumena after extrusion of the body. In another embodiment, the detectible component is a metallic strip of radiopaque material, such as tantalum, which is threaded through one of the secondary lumena. In the case of the remotely detectible component being a material having a different density from that of the body, it can either be injected into the secondary lumen after extrusion of the body or co-extruded with the body.

As stated, the detectible component can be formed integrally with the article or inserted after extrusion of the article into a secondary lumen.
Additionally, the component can extend either substantially along the entire length of the body, coextensive with the primary lumen, or along only a portion thereof. In a particularly advantageous embodiment of the invention, two detectible components are provided and disposed substantially diametrically opposedly in relation to the central, primary, lumen so that the effective flow diameter of the primary lumen can be determined by inspection of the graft.

A method for non-invasively monitoring a patient whose damaged or dysfunctional vascular pathway has been replaced with the present device is also the subject of this further aspect of present invention. The method comprises implanting the inventive device in the patient under conditions sufficient to establish blood flow through the primary lumen. At least one secondary lumen contains remotely detectible material, such as radiopaque or MRI detectible material. The position and/or patency of the graft can thereby be ascertained by examining the patient using a non-invasive technique such as x-ray or fluoroscopic imaging, in the case of radiopaque materials, or MRI or ultrasonic scanning, in the case of differing density materials.

In accordance with yet another aspect of the invention there is provided an implantable prosthetic device for sustained release of a bioactive material into a fluid flow pathway of a patient. The device comprises A body formed of material suitable for implantation which defines a multiplicity of capillary lumena. The body is adapted for connection to the patient's fluid flow pathway to establish fluid flow through the capillary lumena. The lumena are separated by walls, formed by the body, which are sufficiently permeable to allow translumenal permeation of a bioactive material.

The inventive prosthetic device acts as an artificial organ for accommodating various types of fluid flow. For example, the device can be grafted to a patient's vascular system whereby it will act as an artificial blood vessel for transporting blood flow. The device can also be implanted in a patient, however, to transport flow of other types of bodily fluids. By seeding selected lumena of the device with a bioactive material, such as a therapeutic agent, diagnostic agent, or cultured cell type, for contact with the body fluid, such as blood, the fluid can be treated by its passing through the device. For example, by seeding selected lumena with liver cells and connecting the device with the patient's vascular system, an artificial organ for detoxifying blood is formed.

While in a preferred embodiment of this aspect the lumena defined by the implantable body are substantially equal in diameter, in some applications the lumena may be of unequal diameter. In any case, the lumena will typically be of an internal diameter between approximately 0.5 mm and 6 mm, depending upon the outer diameter of the body and the anatomical application for which the device is being used.

In a particularly advantageous embodiment of the invention, interior surfaces of the lumena are coated with a bioactive agent which can be released into biological fluid flowing through the lumena.

Recently published evidence indicating that cellular activities are controlled by receptors, "molecular switches" on the membrane surface of cells, suggests that bioactive and pharmaceutical drug interactions, whether initiators or inhibitors, can be utilized to improve implantable organ and autogenous organ transplant performance. These receptors control cellular activities by binding with highly specific substances referred to as "ligands." Like the action of a key in a lock, ligands fit into receptors and, if the fit is precise, turn on or off certain cellular processes. Some ligands act as antagonists to inhibit cellular activities by blocking a receptor. Research has shown that many -aspects of-cardiovascular disease are controlled by specific cell surface receptors.

It is a feature of the invention, therefore, to provide site specific drug delivery to an affected area for either initiator or inhibitor drug treatment. This is facilitated by the implantable organ which allows blood or other body fluid to flow through it and to contact or pick up an agent such as, for example, a specific protein, drug, enzyme, or antibody being secreted by the multiple lumenal flow surfaces of the implantable device.

Depending upon the site of the implant, each anatomical application may require totally different pharmacological agents as well as different rates of diffusion across the blood contacting barrier from site to site. Each anatomical site may require replacement from time to time, depending upon the drug or combination of drugs required. Regardless of the application or biological activity, however, the basic principle of passive drug diffusion across a lumenal microporous membrane into a flowing body fluid, remains the same.

Since various combinations of antiproliferative agents are known to be effective inhibitors of intimal hyperplasia, it is another feature of the invention to provide effective site-specific delivery of these antiproliferative agents. This feature helps to prevent stenosis and restenosis of the implantable device of the invention.

Typically, the implantable device of the invention is cylindrically shaped. It can, however, be adapted to other profiles for given applications. For example, the body can be extruded to be D-shaped with a flat or flattened surface, so as to have a low profile. This may be desirable where the implantable device is to be implanted just under a patient's skin and needs, therefore, to be unobstrusive.

Various extrudable materials are suitable for forming the implantable body of the invention. In particular, expanded polytetrafluoroethylene (PTFE) has been found to be well suited for the present invention. When using PTFE for this purpose, as described in greater detail herein below, a paste of the material is extruded in the basic form of the implantable device which is then expanded at a specified rate to create an interrelated network of nodes and fibrils.

The invention also features a method for providing a bioactive material to a patient comprising the steps of treating the interior surfaces of the lumena of an implantable device with a bioactive material as described above and implanting the device in a patient so that the patient's bodily fluids pass through the lumena and come into contact with the interior surfaces. Typically, the device is implanted so that it is in fluid communication with the patient's arterial or venous system. Additionally the device can be used to create an arterial-venous shunt for hemodialysis or vascular access. Other methods of implantation, such as intra-organ implantation, can be utilized as well.

Various methods that are generally known in the art can be used for treating the interior surfaces of the lumena with bioactive materials. In one embodiment such treatment may consist of entirely filling selected lumena with a biologically active material or pharmaceutical matrix for diffusion through the walls and into the adjacent lumena which contain body fluid, such as blood. In another embodiment, the interior surfaces may be coated with such a material, or may be coated or seeded with cells that are to incubate and culture within specific lumena or the prosthesis. This may involve first coating or modifying the surface with glycoproteins such as fibrinection or pretreatment with plasma polymerization application, followed by seeding of the surface with a desired cell type, such as autogenous or genetically enhanced endothelial cells, islet of langerhorn pancreatic cells, or those of a particular organ.

In another example of use of the invention, autogenously derived cultured cells and genetically engineered cell complexes which produce and secrete organ specific proteins, enzymes, initiators, or inhibitors can be seeded in the lumena for introduction to a body fluid flow pathway. For example, cultured cells which incubate within the lumena can be injected into the lumena prior to implantation, to produce a desired pharmaceutical agent for diffusion across the lumenal fluid contacting surface. Also, cultured cells to grow through the microporous lumenal membrane and actually line blood contacting surface for direct cell to blood contact and resultant receptor/inhibitor stimulation, and/or secretion.

In accordance with yet another aspect the present invention which may be implemented separately or in addition to the above-recited aspects, a vascular prosthesis comprises plural longitudinally parallel tube structures which are attached to one another over at least a portion of their longitudinal extent. Each of the tube structures comprises a wall defining a longitudinally extending biocompatible exterior surface and a lumen of predetermined diameter for channeling fluid flow therethrough, and the parallel tube structures are releasably attached to one another at their exterior surfaces. The prosthesis is formed to permit manual spatial separation of the tube structures. That is, the structures can be physically separated, at least partially, to form a branched tubular structure. For example, depending on the number of tube structures forming the prosthesis, therefore, a bifurcated, trifurcated, or other branching tubular structure can be formed.

The lumen of each tube can differ in diameter to allow separation of the tubes into, e.g., arterial grafts and venous grafts having different diameters. The lumena can form separate and distinct flowthrough paths along the entire longitudinal extent of the prosthesis, or can join at one end to form a single lumen. Various embodiments of the invention are adapted to optimize specific implant requirements.

In a preferred embodiment of this aspect of the invention, the tube structures are manufactured from stretched and/or expanded polytetrafluoroethylene (PTFE) using a coextrusion process. The structures can optionally include an identifying indicia, such as colored lines, to distinguish each structure from the others.

This vascular graft has several advantages. For example, the amount of surgical time it takes to implant a vascular graft is determined, in part, by the amount of time it takes a surgeon to create each anastomosis. Using the device of the present invention, the number of anastomoses, thus surgery time, is reduced because for a many-to-one junction, the common end of the branches only has to be sutured once. That is, the graft of the present invention requires a single connection to a patient's vascular system at its proximal end, followed by a connection for each distal end. By contrast, using a plurality of single stranded grafts, each graft requires at least two anastomoses, one at the point of origin and another at the point of insertion. The time saved by use of the present invention, therefore, can be significant.

In addition, the ability to implant multiple grafts with a single originating anastomosis allows surgeons to effectively perform multiple grafts which follow the branching of the natural vascular pathway using the inventive prosthetic device. The present invention allows a branched arterial or venous graft to be implanted without the necessity of suturing two grafts together. In various other embodiments of the invention, the structure can be formed so that the various lumena have specific diameters effective either to provide equal portions of blood flow from a single vessel, or to divide the flow in different relative proportions. The structures may further be formed to provide a limit to the degree of separation of the tubular structures.

The present device and method have several advantages, including more flexibility than vascular graft structures having a two-piece coaxial or multi-layer design. The presence of a polymer material completely surrounding the graft can inhibit blood flow through the graft lumen, and increase the rigidity of the grafts. The present structures provide grafts which more clearly approximate the behavior of naturally occurring veins or arteries.

### Brief Description of the Drawings

The foregoing and other objects of this invention, the various features thereof, as well as the invention itself, may be more fully understood from the following detailed description, when read together with the accompanying drawings, in which:

FIGURE 1A is a schematic perspective view of a bilumenal vascular prosthesis of the present invention.

FIGURE 1B is a schematic longitudinal cross-sectional view of the embodiment of FIGURE 1A.

FIGURE 1C, 1D and 1E show schematic cross-sectional views of alternative configurations of the bilumenal device of FIGURE 1A.

FIGURE 2 shows a schematic front elevation view of a die used in the manufacture of the bilumenal device of FIGURE 1A.

FIGURE 2A and 2B show schematic cross-sectional views of the die of FIGURE 2 taken along planes A-A an B-B, respectively.

FIGURE 3 is a schematic side elevation view of a bilumenal device in which the secondary lumen is partially co-extensive with the primary lumen.

FIGURE 3A is a schematic cross-sectional view of the embodiment of FIGURE 3.

FIGURE 4 is a schematic perspective view of a multilumenal device of the invention.

FIGURE 5 is a schematic longitudinal cross-sectional view of an embodiment of the invention having a preattached mini-pump.

FIGURE 6A is a schematic perspective view of an alternate multilumenal embodiment of the invention.

FIGURE 6B is a schematic perspective view of an alternate configuration of the embodiment of FIGURE 6A.

FIGURE 7 is a schematic perspective view of an alternate configuration of the multilumenal embodiment of FIGURE 6A.

FIGURE 8 is a schematic perspective view of a self-sealing vascular tubular structure.

FIGURE 9 is a cross-sectional view taken along line 9-9 of FIGURE 8.

FIGURES 10A and 10B are schematic cross-sectional views of other embodiments of the self-sealing vascular tubular structure of the invention.

FIGURES 11A, 11B and 11C show an exemplary die for manufacturing by extrusion the vascular tubular structure of the invention.

FIGURE 12 is schematic illustration showing a perspective view of an implantable bilumenal vascular graft having a radiopaque material integrated in one of the lumena.

FIGURE 13 is schematic illustration showing a perspective cutaway view of the vascular graft shown in FIGURE 12 showing both lumena without the radiopaque material.

FIGURE 14 is schematic illustration showing a perspective view of a trilumenal vascular graft of the present invention in which the two small lumena --both contain a radiopaque material.

FIGURE 15 is schematic illustration showing a perspective view of a quadrilumenal embodiment of the vascular graft of the present invention in which the three small lumena all contain a radiopaque material.

FIGURE 16 is a perspective view of one embodiment of a polylumenal prosthetic device constructed in accordance with the teachings of the present invention.

FIGURE 17 is a cross section view taking along line XVII-XVII of FIGURE 16.

FIGURE 18 is a perspective view of another embodiment of the prosthetic device of the present invention.

FIGURE 19 is a perspective view of still another embodiment of the prosthetic device of the present invention.

FIGURE 20 is a perspective view of yet another embodiment of the prosthetic device of the present invention.

FIGURES 21A and 21B are schematic views of a die suitable for extruding a polylumenal prosthetic device constructed in accordance with the teachings of the present invention.

FIGURE 22 is a schematic perspective view of a bilumenal separable vascular prosthesis with a cap at one end.

FIGURE 22A is a schematic cross-section of the embodiment shown in FIGURE 22 taken along axis A-A.

FIGURE 23 is a schematic perspective view of a bilumenal, separable prosthesis.

FIGURE 23A is a schematic, enlarged perspective view of the notched end of the embodiment of FIGURE 23;

FIGURE 24 is a schematic perspective view of a bilumenal prosthesis attached to a blood vessel.

FIGURE 25 is a schematic perspective view of a bilumenal, separable, vascular prosthesis; and

FIGURES 26-26A are schematic showing a representative dilumenal prosthesis with a perforated divisible wall between the tubes.

Like reference characters in the respective FIGURES indicate corresponding parts.

### Detailed Description of the Invention

In its broadest aspect, the invention features a multi-lumenal prosthetic device for implantation into a patient. The invention can be utilized, for example, as a vascular graft providing sustained release of a selected bioactive agent or diagnostic material directly into a blood or other fluid flow pathway. The device has at least two lumena which are separated by a porous, semi-permeable wall. In the case of the device being used as a vascular graft, it is grafted onto a vein or artery in an individual such that the primary lumen becomes part of the individual's blood flow pathway. The secondary lumen is filled with a material such as, for example, a bioactive or diagnostic agent and the porous wall between the lumena allows the material disposed in the secondary lumen to diffuse into the bloodstream flowing through the primary lumen. As described in further detail below, the device allows the release of the material across the wall or membrane into the bloodflow pathway in a controlled manner.

The device comprises a main, or primary lumen, which is of a diameter sufficient to allow blood flow appropriate for the artery or vein to which it is attached to occur. Thus, the device has the geometric configuration of a tube, open at least at one end, and typically at both ends. The open end is sutured to an opening in the patient's arteriovenous pathway, thus becoming an extension of that pathway.

The device also contains at least one secondary lumen adjacent to the first lumen. At least one of the secondary lumena contains or is adapted to contain the selected bioactive or diagnostic materials. These materials can include, for example, therapeutic or prophylactic agents, such as a drug, protein, enzyme, antibody or other agent, or cells which produce a drug, protein, enzyme, antibody, or other agent. The diagnostic material can include, for example, a radiolabeled antibody or antigen.

In one embodiment of the invention, one or more of the secondary lumena are pre-filled with the bioactive material. For example, the pre-filled secondary lumen can contain cells which secrete a bioactive agent. As a variation of this embodiment, another embodiment may include a pre-filled lumen in conjunction with other secondary lumena into which drugs or diagnostic materials are introduced after implantation.

Specific embodiments of the device are illustrated by the Figures. FIGURE 1A shows a bilumenal tubular structure 10 having a first lumen 12 and a secondary lumen 12'. The first lumen 12 has a structure sufficient for blood flow therethrough. For a vascular graft, the diameter of this lumen 12 is generally the same or similar in size to the host artery or vein to which it is grafted. As mentioned, however, the structure 10 can be formed to accommodate other types of fluid flow.

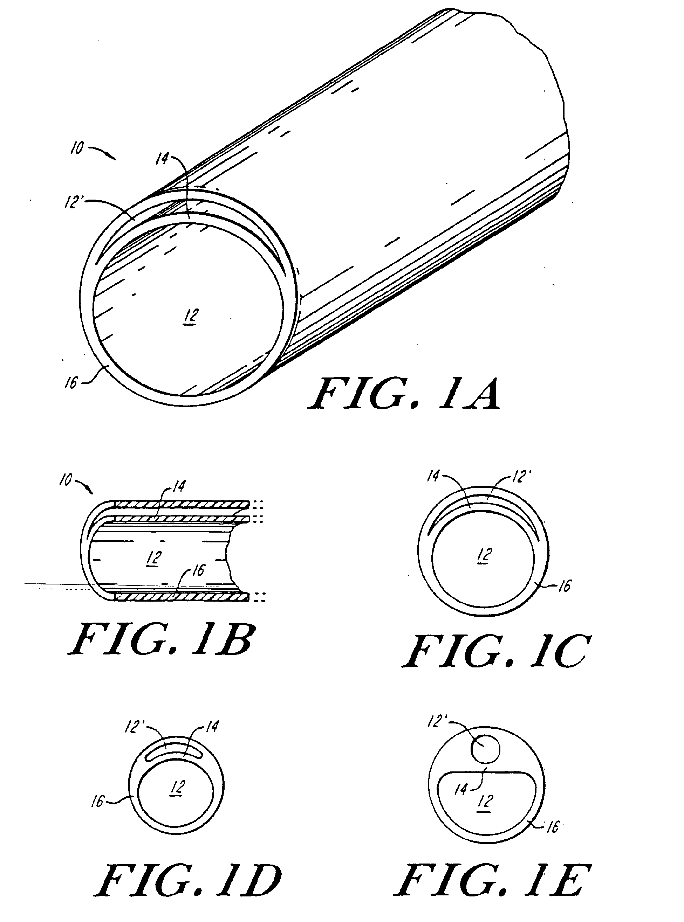

As shown in FIGURE 1B, the secondary lumen 12' is adjacent the first lumen 12, and-is separated by a semi-permeable, micro-porous, wall 14. The wall 14 has a permeability sufficient to allow diffusion of the bioactive agents or diagnostic material of choice from the secondary lumen 12' into the fluid flow pathway defined by the first lumen 12. In a multi-lumen arrangement, i.e., a device having more than two lumena, the adjoining wall 14 lies in communication between each of the secondary lumena and the primary lumen. Alternatively, there can be a semi-permeable wall between each secondary lumen 12' and the first lumen 12, with an impermeable wall among the secondary lumena.

The thickness and permeability of the wall 14 can be adapted to accommodate different drugs, bioactive or bioinert agents, and the like, and to control the rate at which the material disposed in the secondary lumena 12' diffuses across the wall 14. Control over the release of the drug or agent can be obtained by choosing appropriate molecular weights, degrees of crystallinity and/or expansion parameters in the polymer matrix forming the structure 10. The wall 14 can be manufactured having a predetermined permeability factor, and the concentration of materials to be transported across the membrane can be selected accordingly.

The opportunity to control the permeability of the wall 14 is afforded during the extrusion and expansion process as discussed in greater detail below. By controlling the intermodal distance of the polymeric matrix and properly selecting the specific resin composition and expansion conditions, the porosity of the wall 14 can be determined.

The outer diameter (OD) of the external wall of the structure 10 is generally in the range of from about 3 mm to about 30 mm. The internal diameter (ID) of the primary lumen is generally from about lmm to about 28 mm depending upon the type of blood flow through the prosthesis. The OD and ID vary according to the type of pathway for which the prosthesis is used. For example, arterial prostheses will generally have OD of from about 6 mm to about 18 mm, whereas venous prostheses will generally have an OD of from about 12 mm to about 24 mm. The ID of the secondary lumena 12' is generally from about .1 mm to about 6 mm, depending upon the OD.

As shown in FIGURES 1C, 1D, and 1E, the cross-sectional configuration of the lumena designed in accordance with the invention can vary in size and shape depending upon the specific application. In FIGURE 1C, the secondary lumen 12' extends over approximately one-third of the circumference of the lumen 12. FIGURE 1D shows a diminished relative size of the secondary lumen 12' with respect to the first lumen 12, and FIGURE 1E shows a first lumen 12 having an adjacent secondary lumen 12' of dimensions sufficient only to transport substances of a few microns in size. The relative size of the secondary lumen 12' depends on the type of substance to be transported through secondary lumen 12', and the desired rate of diffusion across wall 14. The permeability of wall 14 may be a factor in determining the appropriate size of secondary lumen 12'.

In another aspect, the invention features a method for delivering a bioactive agent or diagnostic material directly to a patient's body fluid, bloodstream or otherwise, in a controlled manner. In the method, a surgeon or other qualified person, surgically exposes the desired-region of the patient for introduction of the prosthetic device 10. The desired site may, for example, be an area of occlusion or weakness in the patient's arteriovascular system. In such a case, during interruption of the patient's blood flow the prosthesis 10 is surgically implanted and sutured or otherwise secured in place. Proper positioning of the prosthesis 10 requires alignment of the primary lumen 12 with the blood flow pathway such that the blood flow is diverted through the primary lumen 12. The secondary lumen 12' either contains or is filled with a drug or agent of choice. The drug or agent perfuses across the interlumenal wall 14 into the bloodstream at a controlled and substantially continuous rate. In this manner, the present invention allows continuous administration of the drug over a prolonged period of time similarly to controlled release systems which deliver a drug at a predetermined rate over a definite period of time.

In an example of use of the present invention, an antibody which is specific to a protein indicative of the presence of malignancy is introduced into the vascular system of a patient utilizing the present device and method. The device 10 is surgically implanted in a patient's vascular system and blood flow is established through the primary lumen 12. The antibody is added to the secondary lumen 12' either before implantation by pre-filling the lumen or after implantation by injecting the antibody composition into the secondary lumen. The antibody is labelled, for example, with a remotely detectable radioisotope such as 125_{I}. The labelled antibody moves across the porous wall 14 between the primary-lumen 12 and the secondary lumena 12' into the patient's blood flow pathway, which flows-through the primary lumen 12. Once in the pathway, the labelled antibodies travel to the site of the malignancy and bind at the appropriate protein binding sites. The concentration of the radioisotope at the target site can then be detected using an appropriate detection device, e.g., a gamma camera. Additional doses of the antibody composition can be injected into the secondary lumen 12' through a cannula, or through a pre-attached catheter which communicates with the secondary lumen 12', without directly invading the patient's bloodstream.

In another exemplary use of the present invention, combinations of anti-coagulant platelets and plasminogen activators, such as heparin, hirudin and tPA can be injected into the secondary lumen 12' to be released at a controlled, continuous, rate into the bloodstream. Continuous release of a solution containing either heparin, tPA, or combinations of the two into a vascular graft area has been shown to be effective in reducing occlusion from intimal hyperplasia, while not resulting in whole body systemic anti-coagulation. Clifton et al., Heart & Lung, 199(1): 115-118 (March, 1991).

In another example of use of the invention, the prosthesis 10 can be used for distribution of chemotherapy agents which are often very toxic and often cause arterial and venous blood vessel destruction at the needle catheter entry site. A major complication of such chemotherapy is the constant risk of permanent, significant delivery site complications such as infection or occlusion due to thrombus formation at the vessel wall injury. The high concentration of such toxic agents very often has a deleterious effect on the blood vessel, further complicating the healing required for the needle tract or catheter injury incurred at the site of drug infusion.

The device 10 eliminates such needle and catheter injury to the blood vessel and large bolus chemical exposure to the native vessel with these highly toxic chemotherapy agents. Moreover, the device 10 can be replaced if long term therapy causes eventual failure or thrombus occlusion. A vein or artery permanently destroyed by chronic therapy, on the other hand, is irreplaceable. Similar problems and corresponding solutions also exist for hemodialysis patients whose native veins are consumed by repetitive needle penetration and permanent blood vessel wall injury.

The secondary lumen forming a chamber or reservoir for a drug or other bioactive substance need not be fluid-filled, but may be filled with a solid or powder, which,upon perfusive exposure to surrounding fluids, releases its active agent into the fluids. In one aspect of the invention, a substance is incorporated in a binder that is drawn or extruded to form a wire or rod of active materials, which is then cut to length and threaded into the secondary lumen. Perfusion rate and dose are carefully controlled by selection of a binder material having the desired in vivo solubility and of a wire gauge, selected to determine a desired total amount of exposed surface area. In addition, the solid wire or rod is cut to length, to determine the total dose. The provision of medications in a leachable or resorbable wire body in this manner is thus seen to offer great simplifications over the prior art techniques of medication or coating a prostheses, which generally involved lengthy steps of soaking and conditioning the graft material.

A method of practicing this aspect of the invention includes providing a vessel or organ graft prosthesis surgically implanted and spliced into a vessel or organ, the graft prosthesis having one or more included chambers in the form of lumena extending along the graft, and providing one or more string, rod or wire shaped solid pieces of a treatment material in one or more of the lumena to locally perfuse the material into the graft over an extended time.

The tube structures of the invention can be manufactured from any suitable biocompatible material that can be arranged to form a microporous structure. Polymeric materials which are useful for this purpose include, for example, either expanded or unexpanded polytetrafluoroethylene (PTFE), Dacron® brand polyester, and other synthetic polyester fibers such as mandrel spun polyurethane and silicone elastomeric fibers. Also, copolymeric materials such as described in U.S. Patent Nos. 4,187,390 and 4,973,609 can be utilized. These are materials made up of more than one type of monomer and have advantages as described in the cited patents, in some applications. The structures can also be formed by extrusion, form molding, or weaving using techniques well known in the art.

In a preferred embodiment, the inventive prosthesis is manufactured by paste forming and rapidly stretching and/or expanding highly crystalline, unsintered, polytetrafluoroethylene. Paste forming by extrusion of PTFE is well-known in the art. Generally, the steps in paste-forming include mixing the resin with a lubricant, such as odorless mineral spirits, and then forming the resin by extrusion into shaped articles. The lubricant is removed from the extruded article by drying, following which the article is sintered by its being heated above its crystalline melting point of approximately 327°C. The sintered, unexpanded, article is a relatively impermeable product. To achieve a greater degree of permeability in the finished product, however, the prostheses of the invention can be formed from an unsintered resin.

Paste-formed, dried, unsintered, shapes can be further treated by expanding and/or stretching them in one or more directions under certain conditions so that they become porous yet retain their strength. Such stretching and expansion with increased strength occurs with certain preferred tetrafluoroethylene resins, e.g., PTFE. The porosity of the material is affected by the temperature and rate at which it is stretched and expanded. A method for manufacturing porous PTFE tubing appropriate for use in the present invention is described in detail, for example, in U.S. patent 3,953,566, and U.S. patent 4,973,609 the teachings of both of which are hereby incorporated by reference herein.

Stretched and expanded PTFE is characterized by a microstructure of nodes interconnected by small fibrils. The space between the nodes and the number of fibrils is controlled by changes in the temperature and rate of stretching and expansion of the PTFE, to produce tubing having predetermined porosity and flex qualities. For example, products which are stretched and expanded at high temperatures and high rates have a more homogeneous structure, i.e., they have smaller, more closely spaced nodes, which nodes are interconnected with a greater number of fibrils. While the resulting structure is stronger than products stretched and expanded at lower temperatures and rates, the porosity is also reduced. Thus, by controlling these two factors, it is possible to construct a series of tube structures having a range of porosity within a desirable range of strength.

Tube structures manufactured as described above begin to lose their crystallites and the crystallinity decreases above this temperature. This is accompanied by a concomitant increase in the amorphous content of the polymer. Amorphous regions within the crystalline structure greatly inhibit slippage along the crystalline axis of the crystallite and lock fibrils and crystallites so that they resist slippage under stress. Heat treatment may be considered to be, therefore, an amorphous locking process, which results in an increase in the amorphous content and of the strength of the treated structure. Heat treatment above 327°C has been found to cause a two-fold increase in the strength of PTFE is approximately 345°C, heat treatment is even more effective. Similar results can be achieved at lower temperatures if expose time is accordingly increased. The optimum heat treating temperature is generally in the range of from about 350°C to about 370°C, with heating periods in the range of from about five seconds to about one hour. Other factors upon which the strength the polymer matrix is dependent upon include the strength of the extruded material before expansion, the degree of crystallinity of the polymer, the rate and temperature at which the expansion is performed, and amorphous locking.

The tube structures of the invention can be formed using other paste-forming operations known to those skilled in the art, for example, any of the available molding processes. Resins other than PTFE may also be used which are generally formable into such tube structures, and which may result in relatively fluid impermeable structures.

The prosthesis 10 can be coated on both the interior lumenal and/or the exterior surfaces with a biocompatible material to render it more hydrophilic or hydrophobic, or to allow specific protein binding or attachment after implantation. Coating materials which are useful for this purpose include, for example, various glycoproteins, albumin or polymeric coatings often used for plasma polymerization, and solvable polymeric coatings such as EVA and PVA Due to the physiological properties of both the arterial and venous system, however, it is important for the prostheses 10 to be gas permeable, or selectively gas permeable, to permit oxygen-carbon dioxide exchange. However, even gas impermeable tube structures may be useful as vascular grafts in certain anatomical applications.

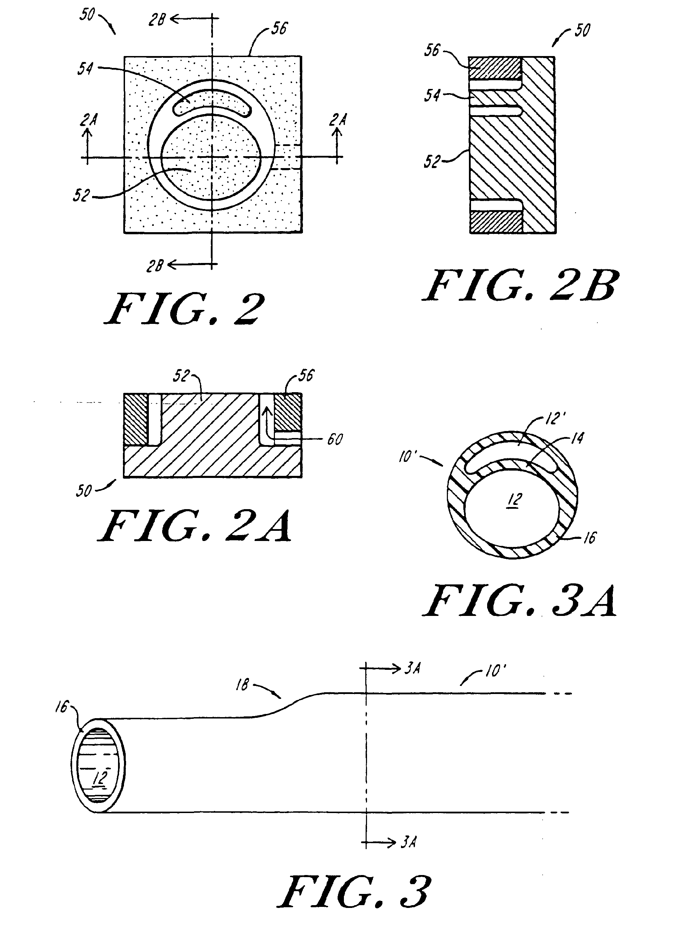

As stated, in the preferred embodiment, the prostheses of the present invention are formed by extrusion of PTFE which is performed using dies of predetermined shape of the type known in the art. FIGURES 1C through 1E, for example, show cross-sectional views of prostheses of the invention made using different exemplary dies. In particular, FIGURE 2 schematically shows an exemplary die 50, corresponding to the illustrated prosthesis of FIGURE 1A. The dies are manufactured from materials and according to methods which are well known in the art.

Generally, and as is illustrated in FIGURE 2, the die 50 consists of a peripheral support structure 56 encasing a first solid die-piece 52 for forming a first lumen, and a second solid die-piece 54 proximal to the first die-piece 52 for forming a secondary lumen. The specific spacing of the first die-piece 52 from the second die-piece 54 depends on the specific desired prosthesis configuration. As best shown in cross-section in FIGURE 2A, the die 50 may include an external port 60 for introduction of PTFE paste or the like for extrusion. FIGURE 2B shows in cross-section the exemplary die 50 of FIGURE 2, showing aperture 58 for forming membrane (14 of FIGURE 1) of the invention.

After the PTFE resin is formed, such as by extrusion as discussed above, it is stretched and/or expanded and then sintered while being held in the stretched and/or expanded state. Stretching refers to elongation of formed resin while expansion refers to enlargement of the formed resin perpendicularly to its longitudinal axis. The rate of stretching and the stretch ratio affect the porosity of the finished product in a predictable manner allowing a prosthetic device to be produced having a specified porosity. The rate of stretching refers to the percentage of elongation per second that the resin is stretched while the stretch ratio refers to the relationship between the final length of the stretched resin and the initial length of the stretched resin. For example, stretching an extruded PTFE tube at a stretch ratio of two to one and a stretch rate of sixty results in a porosity of approximately forty. This porosity is unitless and is determined as set forth on page eighty-four of the American Society For Testing of Materials' Special Technical Publication Number 898. So, for example, based on stretch ratios ranging from two to one, to six to one, a stretch rate of sixty percent per second yields a porosity of between approximately forty and approximately ninety, a stretch rate of one hundred and forty percent per second yields a porosity of between approximately sixty and approximately eighty-five, and a stretch rate of nine hundred percent per second yields a porosity of between approximately sixty-five and approximately eighty-five.

In addition to the porosity, the geometry of the node and fibril network of PTFE can be controlled during stretching and expansion. In the case of uniaxial stretching, that is, elongation of the formed PTFE resin along the direction of extrusion, the nodes are elongated causing the longer axis of each node to be oriented perpendicularly to the direction of stretch. Accordingly, the fibrils are oriented parallel to the direction of stretch. Axial stretching, additionally includes expanding the PTFE resin in the radial direction and can be utilized to produce a prosthetic device having a composite porosity. As in uniaxial stretching, the rate and ratio of radial expansion affects the resulting porosity of the prosthetic device.

In one embodiment, the apparatus of the present invention includes co-extruded plural lumena, the secondary lumena 12' of which extend substantially along the entire length of the first lumen 12. In another embodiment of the invention, however, as illustrated in FIGURE 3, the secondary lumen 12' extends along only a portion of the first lumen 12.

The illustrated prosthesis 10' of FIGURE 3 is structurally similar to the prostheses 10 described above. As shown in cross-section FIGURE 3A, the portion of the prosthesis 10' which includes a secondary lumen comprises a microporous, semi-permeable, wall 14 separating the secondary lumen 12' with the first lumen 12. However, in this illustrated embodiment, the secondary lumen only extends along a predetermined portion of the prosthesis 10'.

The embodiment shown in FIGURE 3 may be manufactured in a manner similar to that described above. The extrusion die for forming the partially-extending lumen may be modified in a manner known to those skilled in the art. For example, the die used in the extrusion of this embodiment of the invention may include a gate-type device which enables selective opening and closing of an aperture to coextrude a secondary lumen.

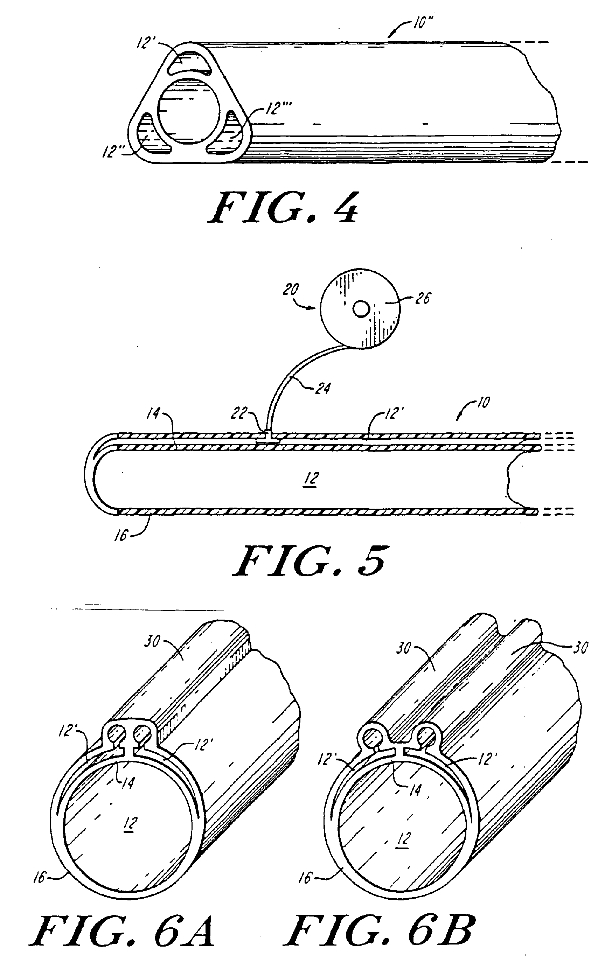

An alternative embodiment of the prosthesis of the present invention is illustrated in FIGURE 4. In the illustrated prosthesis 10', the first lumen 12 is essentially round, with the secondary lumen 12' forming a polygonal configuration around the first lumen 12. While this illustrated embodiment shows three secondary lumen 12', other forms of the invention may include fewer or more secondary lumena. In addition, the apparatus of the invention may have various overall geometric configurations, depending upon the anatomical destination of the prosthesis 10'.

An important aspect of the invention is the introduction of a bioactive agent, pharmaceutical, chemotherapy agent or diagnostic material into a secondary lumen for perfusion into the blood flow pathway of the patient as it is channeled through the first lumen of the prosthesis. This introduction of material may occur by including a mini-pump attached to the secondary lumen of the prosthesis, for example, by a catheter.

As shown in FIGURE 5, a mini-pump 20 may be placed in communication with at least one of the secondary lumen 12' for perfusion of a drug into the secondary lumen 12'. The mini-pump 20 can be used to deliver a predetermined amount of a drug at a preselected rate to the secondary lumen 12. Thereafter, the drug perfuses across the microporous, semi-permeable, wall 14 and_into the bloodflow pathway of the first lumen 12. Mini-pumps which may be used with the prosthesis of the invention are commercially available. Some examples are Alza Pump, Thermedics' Infusaid Pump, Medtronic's Infusable Pump, and INFU·DISKS™ (Electrochemical Drug Delivery, Inc. San Diego, California) designed for subcutaneous delivery of drugs or diagnostic agents. The mini-pump 20 may be located externally to the patient, or may be surgically subcutaneously implanted.

As shown in FIGURE 5, the mini-pump 20 contains a drug reservoir 26 from which a connector tube 24 extends. The connector tube 24 may either be integral with the reservoir 26, or mechanically, detachably connected to the reservoir 26. The connector tube 24 extends from the reservoir 26 to a secondary lumen 12' of a prosthesis 10. The connector tube 24 may connect to the secondary lumen 12' by means of a mechanical attachment device 22, as illustrated, or may be formed integral with the secondary lumen 12'. Mechanical attachment devices are well known in the art, and include luer-locks. It is generally preferable that the mechanical attachment device is such that the mini-pump may readily be replaceable, thus attachment devices which do not require the application of pressure are preferable over pressure-lock devices.

Similarly, a mechanical pump, infusion system, or other drug delivery system located outside the host body may be attached for the delivery of a drug or other bioactive material into a secondary lumen. The drug delivery source may either be integral with the secondary lumen, or may be mechanically, releasably attached.

Alternate embodiments of the present invention are shown in FIGURES 6A and 6B. In those embodiments, the secondary lumena 12' form a raised platform 30 to facilitate subcutaneous needle access. Reservoirs 32 extend from the secondary lumena 12' along the sides of the first lumen 12 to provide a greater surface area for diffusion of the drug across the microporous, semi-permeable, wall 14. As shown in FIGURE 6B, needle access ports 34 may be separated for extracutaneous identification of the individual secondary lumena. In this manner, separate drugs may be delivered to separate secondary lumena.

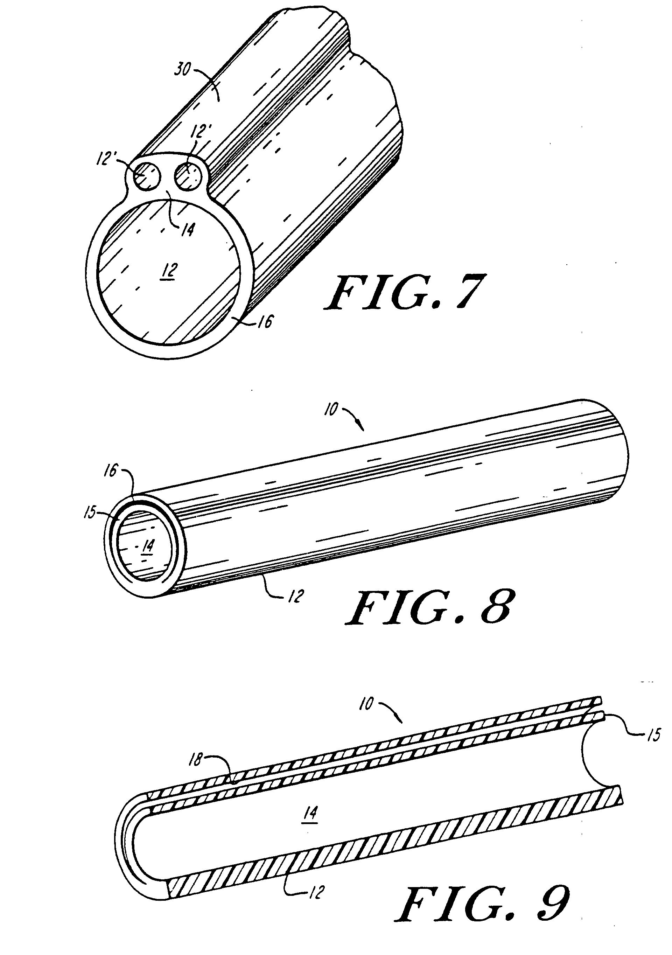

Another embodiment of the prosthesis of the present invention is shown in FIGURE 7, wherein needle access ports 34 are located in a single raised platform 30. In this illustrated embodiment, the secondary lumena 12' do not include reservoirs that extend along the sides of the first lumen 12. The secondary lumena of the illustrated embodiment extend either partially or fully along the length of the first lumen.

In accordance with yet another aspect of the invention, illustrated in Figures 8-11, the invention relates to an implantable self-sealing tubular device comprising at least two lumena. The device defines a primary lumen adapted for blood flow therethrough and at least one secondary lumen which contains a non-biodegradable elastomeric material, and which shares a common side wall with the primary lumen.

The device of the invention can be used as a prosthetic vein, artery, pseudo-organ, or other similar anatomical structure. In preferred embodiment, the prosthesis comprises a vascular graft. The device is implanted into the patient's arterial or venous system so that blood is established through the primary lumen.

The secondary lumen is filled with a non-biodegradable elastomer which self-compresses after puncture by a cannula or needle to seal the puncture site. Elastomeric materials which are useful for this purpose include elastomeric polymers and copolymers, including silicone rubbers, polyurethanes, and polyethers. Various fluoropolymers are suitable as well.

One illustrative device embodying this aspect of the invention is shown in FIGURE 8. The device shown is a self-sealing vascular tubular structure 110 formed of a body 112. The body 112 defines lumena 114 and 118 which share common side wall 15. Disposed in the lumen 118 is a self-sealing elastomeric material 116.

FIGURE 9 is a cross-sectional view of the structure 110 in which it can be clearly seen that the body 112 defines lumena 114 and 118. While in FIGURE 9, the lumena 114 and 118 are of equal lengths, it should be understood that the structure 110 will only have a self sealing capability along the length which is coextensive with lumen 118.

In a preferred embodiment, lumen 114 is of a sufficient internal diameter (ID) to allow blood flow therethrough. This means the ID of the lumen 114 will typically be between about 3 mm and about 24 mm depending on the application. Thickness of the common side wall 115 is generally in the range of between about 0.1 mm and 1.2 mm, depending upon the type of blood flow through the prosthesis. The thickness varies according to the type of pathway for which the prosthesis is used.

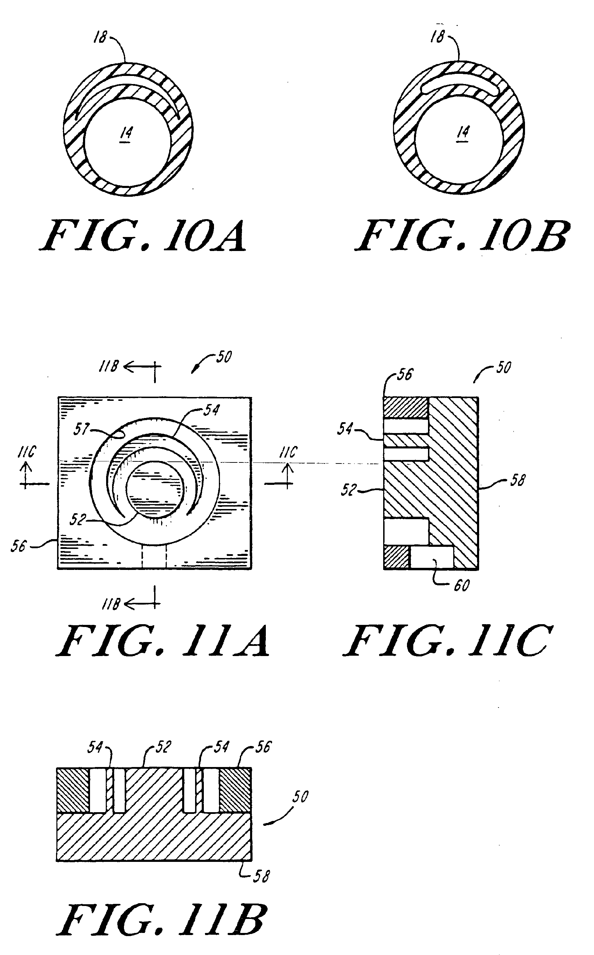

As shown, in FIGURES 10 and 10B, the cross-sectional configuration of the lumena designed in accordance with the invention may vary in size and shape depending upon the specific applications. In FIGURE 10A, the secondary lumen 118 extends over approximately one-third of the circumference of the lumen 114. FIGURE 10B shows a diminished relative size of the secondary lumen 118 with respect to the first lumen 114.

In a preferred embodiment the tube structures of the present invention are formed by extrusion of PTFE. Extrusion is performed using dies of predetermined shape according to principles known in the art. FIGURE 11 schematically shows an exemplary die 150, corresponding to the illustrated prosthesis of FIGURE 8. The dies may be manufactured from materials available and well known in the art.

Generally, as illustrated in FIGURES 11A-11C, the die 150 consists of an upper plate 156 and a lower plate 158. The upper plate 156 defines a circular edge 157 corresponding to the other diameter of the tubular structure 110. The lower plate 158 includes a first hub 154 proximal to the hub 152 for forming a first lumen, such as lumen 114, and a second hub 154 proximal to the hub 152 for forming a secondary lumen, such as lumen 118. The specific spacing of the first hub 152 from the second hub 154 depends upon the specific desired prosthesis configuration. This spacing will dictate, for example, the thickness of the common side wall 115. As best shown in cross-section in FIGURE 11C, the die 150 typically includes a channel 160 for introduction of PTFE paste, of the like, under pressure for extrusion. The manufacture of such dies is understood to be well known in the art.

After the PTFE resin is formed, such as by extrusion as discussed above, it is stretched and/or expanded and then sintered while being held in the stretched and/or expanded state. Stretching refers to elongation of formed resin while expansion refers to enlargement of the formed resin perpendicularly to its longitudinal axis. The rate of stretching and the stretch ratio affect the porosity of the finished product in a predictable manner allowing a prosthetic device to be produced having a specified porosity. The rate of stretching refers to the percentage of elongation per second that the resin is stretched while the stretch ratio refers to the relationship between the final length of the stretched resin and the initial length of the stretched resin. For example, stretching an extruded PTFE tube at a stretch ratio of two-to one and a stretch rate-of sixty results in a porosity of approximately forty. This porosity is unitless and is determined as set forth on page eighty-four of the American Society For Testing of Materials' Special Technical Publication Number 898. So, for example, based on stretch ratios ranging from two to one, to six to one, a stretch rate of sixty percent per second yields a porosity of between approximately forty and approximately ninety, a stretch rate of one hundred and forty percent per second yields a porosity of between approximately sixty and approximately eighty-five, and a stretch rate of nine hundred percent per second yields a porosity of between approximately sixty-five and approximately eighty-five.

In addition to the porosity, the geometry of the node and fibril network of PTFE can be controlled during stretching and expansion. In the case of uniaxial stretching, that is, elongation of the formed PTFE resin along the direction of extrusion, the nodes are elongated causing the longer axis of each node to be oriented perpendicularly to the direction of stretch. Accordingly, the fibrils are oriented parallel to the direction of stretch. Biaxial stretching, additionally includes expanding the PTFE resin in the radial direction and can be utilized to produce a prosthetic device having a composite porosity. As in uniaxial stretching, the rate and ratio of radial expansion affects the resulting porosity of the prosthetic device.

In one embodiment, the apparatus of the present invention includes co-extruded plural lumena, as shown in FIGURE 8, the secondary lumen 118 of which extends substantially along the entire length of the first lumen 14'. In another embodiment of the invention 10', as shown in FIGURE 5, a secondary lumen 18' extends along only a portion of the tubular structure 10' which includes the secondary lumen 18 provides a self-sealing feature.

Otherwise, illustrated tubular structure 10' of FIGURE 3 is structurally similar to the tubular structure 110 shown in FIGURE 8. As shown in cross-section in FIGURE 3A, the portion of the structure 10' which includes a secondary lumen includes a common side wall 15' between the secondary lumen 18' and the main lumen 14'. In the is illustrated embodiment, however, the secondary lumen 18' only extends along a predetermined portion of the structure 10'.

The embodiment shown of FIGURE 3 can be manufactured in a manner similar to that described above. The extrusion die for forming the partially-extending lumen may be modified in a manner known to those skilled in the art. For example, the die used in the extrusion of this embodiment of the invention may include a shunt which allows selective opening and closing of an aperture for coextruding a secondary lumen.

The method of the invention comprises utilizing the present self-sealing graft device to replace or augment part of AV pathway in an individual. In the method, a surgeon or other qualified person surgically exposes the desired region for introduction of the graft of the of the invention. The desired site may be an area of occlusion of weakness in the patient's arteriovascular system, for example. An interruption of the patient's blood flow is performed, and the device is surgically implanted and sutured or otherwise secured in place so that blood flow is established through the primary lumen. Once the graft is in place, the bloodstream can be accessed by a cannula, intravenous needle or the like through the secondary lumen. When the cannula or needle is withdrawn, the elastomer on the secondary lumen will compress thereby prevent blood from escaping from the area of access.

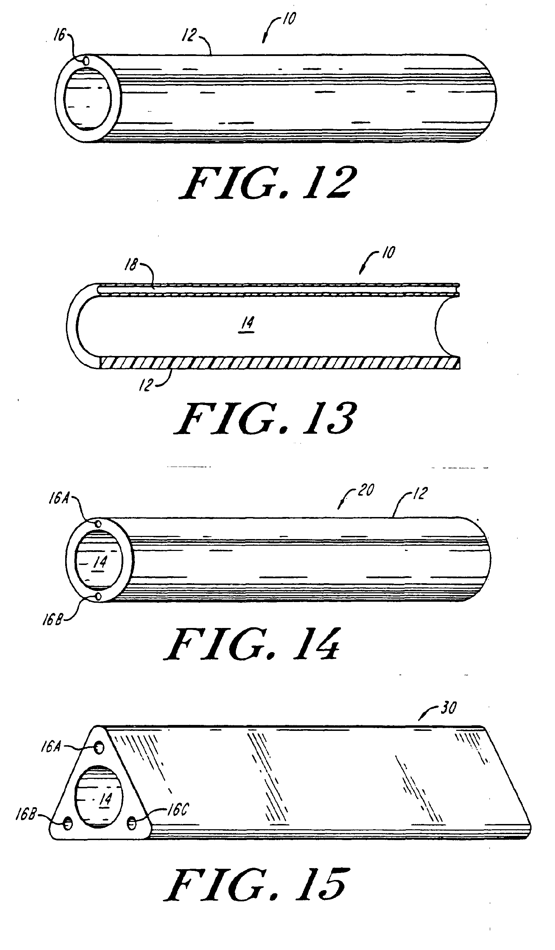

In accordance with the further aspect of the invention shown in Figures 12-15, a prosthetic device such as a vascular graft, includes integral diagnostic indicia for detecting the position or patency of the graft by non-invasive external means. The device is formed from a biocompatible tubular structure which defines at least two lumena, a primary lumen which is adapted for blood flow and at least one secondary lumen within which a remotely detectible component is disposed. The device of the invention allows detection by x-ray, ultrasonic, MRI and/or other non-invasive types of imaging. This allows the position of the graft and/or the degree of patency to be determined.

Various remotely detectible materials which are generally known in the art are suitable for disposition in the secondary lumen. For example, radiopaque materials which are useful in the present invention include barium sulfate and tantalum wire. Also, barium filled elastomers, such as are used for surgical sponges are suitable. Essentially, any biologically inert metal-containing compound can be used. Additionally, the secondary lumen can be filled with a material having a different density from that of the article itself which will enable detection by ultrasonic and MRI techniques.

One embodiment of the present device is shown in FIGURE 12. An implantable vascular graft 210 is formed of a tubular body 212 which defines a lumen 214. Disposed in the perimeter of the article 212 is a remotely detectible component 216 such as those discussed above.

FIGURE 13 shows a cross-section of the vascular graft shown in FIGURE 12, with the detectible component 216 removed. The FIGURE shows an additional lumen 218. The detectible component 216 is disposed in lumen 218. The detectible component 216 can occupy the entire lumen 218 or merely a portion thereof depending upon the requirements of the specific application.

FIGURE 14 shows another embodiment of the present invention wherein an implantable vascular graft 220 includes detectible components 216A and 216B disposed in the graft's body 212. In this manner, by imaging after implantation, the effective flow diameter of the graft 220 can be determined since it is clearly related to the distance between the detectible component 216A and the detectible component 216. In this embodiment of the invention, in addition to the flow-providing lumen 214, the body 212 defines two lumena for accommodating the detectible components 216A and 216B.

Still another embodiment of the invention, shown in FIGURE 15, includes an implantable vascular graft 230 having a body 212 which defines a flow-providing lumen 214 and in which are disposed three detectible components 216A, 216B and 216C. Still other embodiments of the invention will be readily apparent to those ordinarily skilled in the art.

The method according to the just described aspect of the invention is practiced by implanting in a patient a vascular graft as described above to promote flow through a damaged or dysfunctional pathway. In this method, the graft is implanted in a patient such that blood flow is established through the primary lumen of the device. The secondary lumena contain the detectible material. By then examining the graft by x-ray, MRI, or other form of imaging, depending upon the type of material disposed in the secondary lumen, the position, and effective flow diameter of the graft can be determined.

For example, in the case of a graft having a single line of detectible material co-extensive with the graft, the surgeon can follow the line of the graft, e.g., to ascertain whether the graft has become twisted, looped or otherwise not properly positioned during the surgery. In another embodiment, a graft having two or more secondary lumena which are filled with detectible material is implanted. The doctor can then monitor blood flow through the graft by observing through x-ray or MRI imaging, the movement of the detectible lines relative to each other. For example, a fluoroscopic examination by a physician would reveal pulsation of the graft vessel as the detectible lines would constantly move in and out with respect to one another; whereas if blood flow is stopped, no movement will be observed. By using detectible lines which are co-extensive with the graft, the openness of the graft along its entire length can be observed. Another advantage to the present invention is that the detectible material is an integral part of the graft, therefore, it cannot become separated or dissociated from the graft after implantation. The present method of monitoring an implanted vascular graft is, therefore, more reliable than traditional methods involving the suturing of radiopaque markers to a vein, artery, or graft.

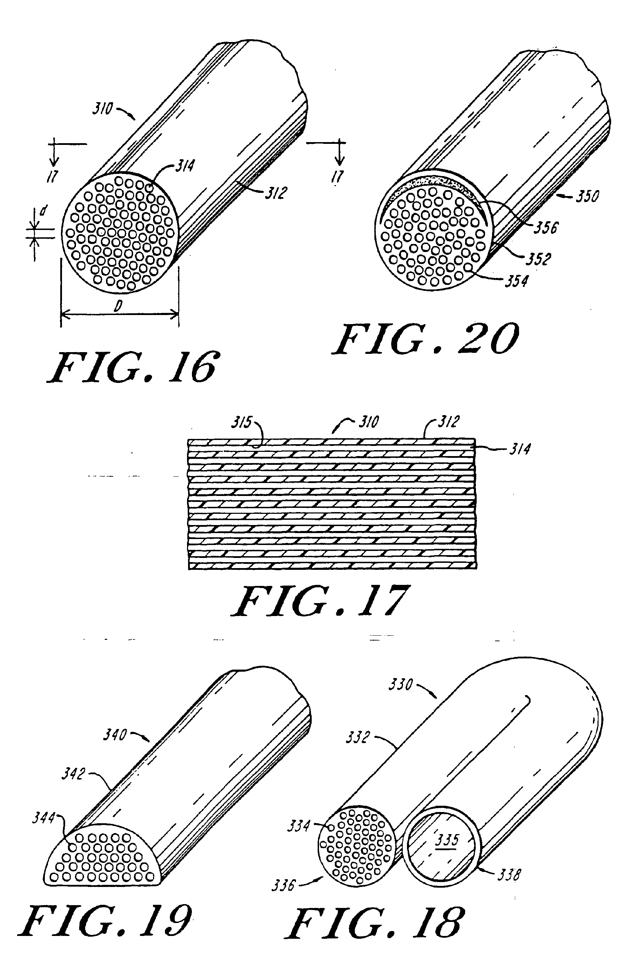

The foregoing description sets forth advantageous properties of a lumenal prosthetic structure for drug delivery, visualization of patency, sealing and other desirable attributes. In accordance with a further aspect of the invention shown in Figures 16-21, the invention features an implantable device formed of a microporous body material which defines a multiplicity of coextruded, molded, or otherwise formed capillary lumena and an outer boundary or wall which circumscribes the lumena. In FIGURE 16, an embodiment of the invention is shown wherein a polylumenal device 310 is formed of an implantable body 312. The body 312 defines a multiplicity of lumena 314 for transporting fluid flow. The outer diameter of the implantable device 310 is indicated as D while the diameter of a typical capillary lumen is indicated as d. Specific values of these characteristics are discussed in greater detail herein below. As described in greater detail below, the device 310 can be formed by any number of paste forming methods such as by, for example, extrusion.

FIGURE 17 is a cross-sectional view of the implantable organ 310 and shows that in this embodiment of the invention the capillary lumena 314 extend the entire length of the device 310 is channeled through the lumena 314 for its entire course. In another embodiment of the invention, however, the lumena 314 do not extend along the entire length of the device 310.

In either case, however, the device 310 has the further property that its structural material has a sufficiently microporous to allow communication of cells and extra-cellular fluid in a direction transverse to the capillary axes. This communication allows cell processes to penetrate the structural material. It also allows the selective trans-lumenal passage of extracellular matter, such as marker chemicals governing cell or organ growth patterns.

In addition to providing a more delicate ultrastructure to encourage cell motility, one advantage afforded by the capillary lumena structure of the device 310 is realized, for example, when the device 310 is utilized as a vascular graft. Increased blood contact surface area 315 makes the device 310 more conducive than known implantable organs having only a single lumenal surface to harboring endothelial cells. This is as a result of the device 310 providing reduced shear pressure on fluid flowing through the lumena 314.

Other advantages of the multiple capillary lumena structure include improved cellular distribution for blood contact and more surface area for supporting the growth of more specialized cell types. Furthermore, the porosity of the body 312 allows perfusion of necessary bioactive and pharmaceutical agents for cell growth, and cell to cell membrane contact necessary for cell metabolism, replication, and subsequent replacement after cell death. Generally, endothelial cell growth is stimulated by several factors, including the binding or adhesive action of glycoproteins such as fibrinection. When the implantable device is precoated with a glycoprotein, therefore, there is an improvement in adhesion of seeded cells, increasing retention and migration of cells during cell growth.

Another advantage of the capillary lumena structure of the device 310 is that relatively small flow channels provide a more desirable shear blood flow characteristic than do large flow channels. As a result, the survival of naturally occurring endothelial cells is enhanced which stays the proliferation of smooth muscle cells into the flow channels. This is significant because, as discussed above, smooth muscle cell proliferation has been clinically identified as a common obstacle inhibiting long term or extended patency of single lumen vascular grafts. By inhibiting the proliferation of smooth muscle cells by creating a more natural flow environment through the capillary lumena 314, therefore, the device 310 is capable of remaining patent for an extended period of time, longer than known single lumen grafts constructed of the same structural material.

For a lumen to accommodate the flow of blood cells, its diameter must be at least approximately 0.5 mm. So, to ensure blood flow while simultaneously providing desirable shear characteristics, the diameter d of the device 310 when used as a vascular graft is preferably between 0.5 mm and 6 mm, depending on the anatomical and fluid flow requirements. As a result, as used herein, the term capillary, when referring to the lumena 314 of the device 310, generally means lumena having an internal diameter ratio of at least 0.5 mm I.D. for O.D.'s of 3 mm, 1 mm I.D. for O.D.'s of 6 mm, and 5 mm to 6 mm I.D. for O.D.'s of 30 mm. It can be seen, therefore, that the ratio of lumena inner diameter to the overall outer diameter of the device is typically approximately one to six.

In addition to encouraging improved harboring of naturally created and/or autogenously cultured and seeded endothelial cells, the increased surface area 315 provides more area for deposition and growth of naturally occurring or seeded genetically enhanced cell types in addition to providing more surface area for bioactive and pharmaceutical fluid and cell interface or contact.

For example, the following chart lists typical pharmaceutical agents appropriate for either coating on the walls of the lumena 314 of the device 310 or filling selected lumena. A combination of the two techniques may be desirable as well. Also included in the chart is the desired effect achieved by the listed pharmaceutical.

| Drug Class | Biological Activity |
|---|---|
| Steroids | Anti-inflammatory, Anti-proliferatory for SMC |
| | |
| Ibuprofen (non-steroid) | Antiinflammatory |
| | |
| Trombin Inhibitors | Reduce Acute Platelet Thrombosus |
| | |
| -Haparin | Antiproliferatory for SMC |
| -Hirudin | Thrombin Inhibition |
| Citrates Antibiotics | Anticoagulant Infection Protection, Prevention, Reduction |
| | |
| Glycoproteins/ Fibronectin | Promotes specific cell attachment to foreign material |
| | |
| Angiotensin | Enzyme Inhibitor |
| | |
| Cyclosporin | Immuno Suppressive Activity |
| | |
| γ-Interferon | Immuno Suppressive Activity |
| | |
| Angiopeptin | Antiproliferatory for SMC |
| | |
| Trapidil | Antianginal Agent, Vasodilator, Antiplatelet Activity |
| | |
| Colchicine | Antimitotic, Antisecretory Property |
| | |
| Dipiridymole | Antiplatelet |
| | |
| Salicylic Acid | Antiplatelet, Inhibits Thromboxane A₂ Production (TXA₂) |
| | |
| PGI₂ (Protaglandin I₂) | Antiaggregation and Vasodilation |
| TRA (Thromboxane Receptor Antagonists) | Blocks TXA₂ Receptors without inhibiting PGI2 production |
| | |
| Nitric Oxide | Relaxes smooth muscle cells, potent antiplatelet aggregating substance |
| Five Types of Plasminogen Activators | Thrombolytic agents - potent clot dissolvers |
| | |
| 1. Steptokinase | |
| | |
| 2. Acylated Steptokinase Plasminogen Activator Complexes (APSAC) | |
| | |
| 3. Urokinase | |
| | |
| 4. Single-Chain Urokinase Plasminogen Activator (scu-PA) | |
| | |
| 5. Tissue Plasminogen (t-PA) | |
| | |
| Combinations of Tissue Plasminogen Activator and Thrombin Inhibitors (i.e., T-PA and Hirudin) | Treatment of acute vascular graft thrombosis, or prevention of same |
| Lovastatin | Antiproliferatory for SMC |
| | |
| Cytotoxic Agents | Various, tumor specific chemotherapy agents |
| | |
| Insulin | For treatment of diabetes mellitus |
| | |
| Beta Blockers | For treatment of arrhythmia |

In FIGURE 18 there is shown an embodiment of the invention wherein an implantable device 330 is formed of an implantable body 332 which defines a plurality of capillary lumena 334. In accordance with the device 330, however, one end 336 defines the capillary lumena 334 while another end 338 defines only a single lumen 335. This embodiment of the invention is well suited for artery to vein grafting wherein the end 336 defining the lumena 334 is connected to an artery of a patient while the end 338 defining only single lumen 335 is connected to a vein of the patient. As a result, the ability of the capillary lumena defining end 336 to prevent arterial steal syndrome is exploited.

Arterial steal occurs when an equivalent size lumen device is bypassed from an originating artery to a lower pressure vein. Under such circumstances, arterial flow can preferentially flow through the bypass device in the vein, thereby reducing flow pressure to the distal destination of the originating artery. Hence the term arterial steal, which is an undesirable effect when creating such arterio-bypass shunts. When a multilumenal device is used for such arterio-venous bypass or shunting, the increased flow resistance of the smaller lumens do not allow unrestricted flow, therefore, preventing arterial steal from occurring.

Another embodiment of the invention is shown in FIGURE 19 wherein a implantable device 340 incorporates a capillary lumena structure 344 circumscribed by an outer wall or boundary 342 having a D-shaped profile. This broader, flatter structure provides better needle access and is less conspicuous in situations in which the device is implanted close to the surface of a patient's skin. Note also that the infra-structure of the device 340 provided by the walls of the lumena 344 provides improved internal strength for confronting the deleterious effects of repeated needle access. That is, the implantable organ of the invention will withstand more needle punctures without aneurysm formation blowout, than well known single lumen vascular grafts which succumb to such undesirable events.

Still another embodiment of the invention is shown in FIGURE 20 which shows a prosthetic device 350 that includes, in addition to a plurality of capillary lumena 354, a larger lumen 356 located just under the surface of the outer wall 352. This larger lumen 356 is suitable for containing, for example, either a self-sealing elastomeric material for providing the device 350 with self sealing capability or a bioactive material such as a pharmaceutical agent. The larger lumen 356 enhances the device's ability to deliver bioactive material to a patient's blood stream through the microporous structure, directly into the blood or body fluids flowing through the multiple capillary lumens.

As noted above, the prosthesis of the invention can be manufactured from any suitable biocompatible material, such as PTFE, Dacron®, or other synthetic polyester, or mandril spun polyurethane or silicone elastomer micro-fibers that can be arranged to form a microporous structure. Hybrid constructions of these same materials are suitable as well. Also, copolymeric materials such as described in U.S. Patent Nos. 4,187,390 and 4,973,609 can be utilized. These are materials made up of more than one type of monomer and have advantages, in various applications, described in the cited patents. The structures may be extruded, form molded, mandrel spun fiber, or woven using techniques well known in the field. In a preferred embodiment, described in further detail below, the implantable device is manufactured from stretched and/or expanded PTFE tubing by rapidly stretching highly crystalline unsintered polytetra-fluoroethylene in one or more planes or axes.

Stretched and expanded PTFE is characterized by a microstructure of large nodes interconnected by fibrils, with the space between the nodes, internodal distance, and the number of fibrils controlled by changing the temperature and rate of expansion of the PTFE to produce structures having predetermined porosity and flex qualities. Internodal distances of from smaller than approximately 0.5 microns to as large as approximately 60 microns are suitable for use with the present invention.

Products which are expanded at high temperatures and high rates have a more homogeneous structure, i.e., they have smaller, more closely spaced nodes, which nodes are interconnected with a greater number of fibrils. While the resulting structure is stronger than products expanded at lower temperatures and rates, the porosity is also reduced. Thus, by controlling the two factors, it is possible to construct a series of tube structures having a range of porosity within a desirable range of strength.

It has been noted that when tube structures, manufactured as described above, are heated to above the lowest crystalline melting point of the PTFE, disorder begins to occur in the geometric order of the crystallites and the crystallinity decreases. This is accompanied by a concomitant increase in the amorphous content of the polymer. So formed amorphous regions within the crystalline structure greatly inhibit slippage along the crystalline axis of the crystallite and lock fibrils an crystallites so that they resist slippage under stress. Heat treatment may be considered to be, therefore, an amorphous locking process, the important aspect of which is an increase in the amorphous content of the treated structure. In fact, heat treatment above 327°C has been found to cause a two-fold increase in the strength of PTFE tubular structures.

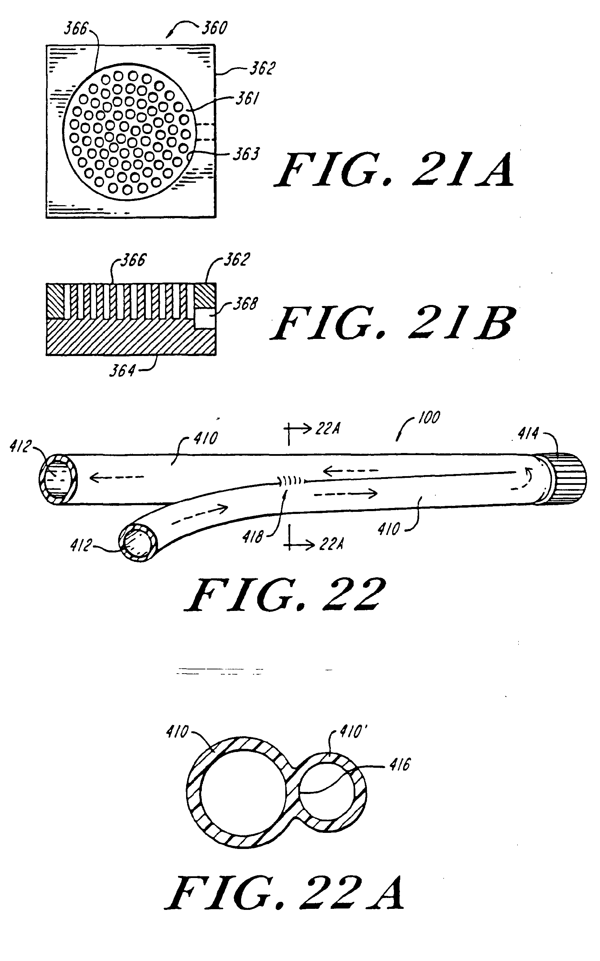

As stated, in the preferred embodiment the tube structures of the present invention are formed by extrusion and expansion of PTFE. Extrusion is performed using dies of predetermined shape which is determined by considerations known in the art. FIGURES 21A and 21B schematically show an exemplary die 360, corresponding to the illustrated prosthesis of FIGURE 16. The die may be manufactured from materials available and well known in the art.

Generally, and as illustrated, the die 360 consists of an upper plate 362 and a lower plate 364. The upper plate 362 defines a circular edge 363 corresponding to the outer diameter of the tubular structure 310. The lower plate 364 defines a plurality of finger-like projections 366 corresponding to the lumena 314 of the prosthetic device 310. The specific dimensions of the projections 366 and their spacing from one another depend upon the specific desired prosthesis configuration. This spacing will dictate, for example, the thickness of the common side walls 315. As best shown in cross-section in FIGURE 21B, the die 360 typically includes a channel 368 for introduction of PTFE paste, or the like, under pressure for extrusion. In practice, the extruded material is supported or carried as it exits the die, so that tensile or compressive forces are controlled and the capillary passages remain open. As discussed above, the material may then be subjected to heat or other treatment for sintering, solvent removal or the like.

After the PTFE resin is formed, such as by extrusion, it is stretched and/or expanded and then sintered while being held in the stretched and/or expanded state as discussed above.

The invention contemplates that in various embodiments the prosthesis may serve not only as a flow structure, but as a matrix and support upon which cellular matter is grown while blood circulates therethrough. For these embodiments, the device may be prepared and seeded with culturable cells, e.g., marrow or organ cells, or endothelial cells which have been genetically modified to produce a desired bioactive material, and may then be implanted in an organ or connected to the AV system. Examples of typical cell types useful for this purpose and their corresponding biologic activities follow.

| Cell Type | Biologic Activity |
|---|---|
| EC (Endothelial Cells) | Endothelial cells line the blood contacting surface of all organs of the body and produce many initiators and inhibitors involved in wound healing, thrombosis, thrombolysis and blood vessel regeneration and cell growth, for direct perfusion into blood stream |
| Islets of Langerhans | Islet Cells produce insulin on demand, based on complex receptor blood sugar chemistry. |
| Pancreatic Cells | directly into the blood stream by the islet cells |

For these embodiments, the spacing between capillaries is preferably small, and may approach membrane thickness in places, to allow communication by direct physical processes or by fluid mediators between cells growing in different capillaries. The body of the prosthesis thus provides a first plurality of capillary channels for direct physical flow of flood for supplying nutrients to living tissue growing on the capillary walls, and a second set of transverse pores or openings of substantially smaller size for communication between cells, so that they may flourish in a three-dimensional matrix. The relatively open or microporous matrix is expected to allow cellular material to grow into micro structures which are similar to those of the structures in which the parent cells naturally occur, thus providing an effective environment for in vivo culture of specialized cell types. As such, it offers the prospect of providing an effective form of organ replacement or supplemental therapy.

In an another embodiment, specific autogenously derived and genetically engineered growth factors can be inoculated into various lumena for body fluid contact. Such growth factors are specific proteins borne by flood platelets that trigger tissue healing.

| Protein Class | Biologic Activity |
|---|---|
| PDGF Protein (Platelet derived growth factor) | Wound healing factor, encourages growth of new blood vessels |
| | |
| EGF (Epidermal Growth Factor) | Promotes wound healing, healing of ulcers reduced degradation and faster recovery of transplanted organs |
| | |
| bFGF (basic fibroblast growth factor) | Signals subdermal tissue growth, promotes blood vessel growth, bone graft growth |
| | |
| TGFb (transforming growth factor Beta) | Promotes wound healing of ulcers caused by varicose veins and diabetic ulcers |
| | |
| Erythropietin | Promotes red blood cell growth |
| | |
| GCSF (Granulocyte colony Stimulating factor) | Promotes white blood cell growth |
| | |
| GMCSF (Granulocyte macrophage colony Stimulating factor) | Promotes white blood cell growth |
| | |
| Iamin (Tripeptide) | Attracts and triggers various cell specific growth factors following trauma |
| | |
| BGF-1 (Heparin-binding growth factor-1) | Stimulates blood vessel growth |
| | |
| CD4 Protein | Controls growth of AIDS virus |

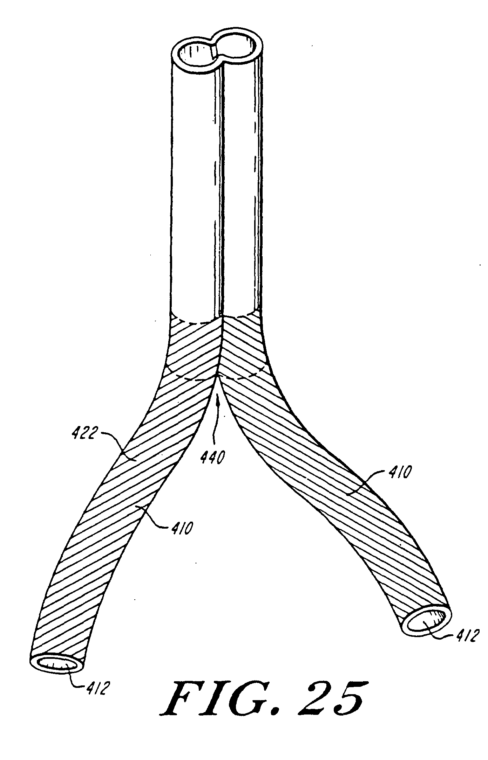

In accordance with a further aspect of the invention shown in Figures 22-26, a vascular prosthesis includes two or more tube structures defining lumena of various diameters for accommodating the flow of fluid, such as blood, therethrough, wherein each of the tube structures is manually separable from the others during surgical introduction of each prosthesis. This allows each tube structure to be used as a separate prosthesis at least one end of each extending to a different vascular site.

In addition to use as a vascular graft, one or more of the lumena may be used for fluid flow of a different nature, such as an organ-to-organ fluid channel, or as a vehicle for drug delivery as described above. The materials used in the construction of the tube structures can be semi-permeable to agents of specific sizes to allow communication of the fluid or drug with the blood flow of an adjacent tube structure. A multi-lumenal vascular graft having drug delivery capability is described in greater detail above, and will not be discussed in further detail here, but it is understood that the present aspect of the invention includes all forms of hybrid vessel/drug delivery combinations, and combinations with other aspects of the invention described above. To prevent osmosis of a drug or agent from a filled lumen to the surrounding tissue, the external surface of the tube structures, or the internal walls surrounding the lumena can be coated with a non-permeable, biocompatible material.

The internal diameter (ID) of each lumen depends upon the intended use for each tube structure. In general, lumena having ID's of from about 3 mm to about 24 mm are useful as vascular grafts. For example, a tube structure intended for insertion in an arterial pathway can have a lumen ID of from about 6 mm to about 18 mm. A tube structure for insertion in a venous pathway can have a lumen ID of from about 12 mm to about 24 mm. The lumena can each have the same or different diameters for insertion into one or more types of pathways. For example, the lumen ID of tube structures to be used as arterial grafts is generally less than the ID lumen for use in venous grafts.

The outer diameter (OD) of the tube structures is generally not related to the internal lumen diameter. For example, in an embodiment of the invention having two tube structures, the external diameter of both tube structures can be the same, while the internal lumen diameter of each tube may differ.

The thickness of the lumen walls will vary depending on the type of vascular graft. Generally, an arterial graft has a thicker wall than a venous graft. However, the exact dimensions depend on the specific purpose. For manufacturing purposes, the die used for extrusion may be varied to achieve walls having the desired thicknesses. Wall thicknesses of from about 0.1 mm to about 1.2 mm are useful for a general range of vascular graft applications.

The tube structures are joined by a wall which allows them to be easily separated. In various embodiments of the invention, the tube structures are separated either by pulling them apart or by cutting them with scalpel or scissors. To facilitate separation, the tube structures are joined by a small bead or seam of material. The bead or seam serves to hold the tubes together while allowing them to be pulled or cut apart without impairing the wall structure at the line of separation. In another embodiment, the tubes are joined by a thickened wall portion which, when separated, forms two walls so that separated tube structures have walls of substantially equal thickness.

In another embodiment of this aspect of the invention, one or more of the tube structures includes identifying markings. These markings can be in the form of lines, dots, or other designs running along the entire or partial length of the exterior surface of each tube structure. These markings distinguish the tube structure from that of the adjacent tube, and may further act as codes indicating, for example, the size of the inner lumen. The different markings allow the user to trace each tube structure should it become twisted during use. The identification markings allow the surgeon, or other user of the invention, to implant the structure at one point along the arteriovenous pathway, then thread it to a distal point while at the same time being able to distinguish one tube structure from the others.

For example, the point of origin for the venous graft may be in the greater saphenous vein, another graft may originate in the adjacent femoral artery, and the point of insertion for the structure is the anterior tibial vein and artery, respectively. In traversing the patient's leg, it would be easy for a surgeon to twist or otherwise lose track of which structure involved venous bloodflow and which structure transported oxygenated blood. Thus, labelling the exterior surfaces of at least one of the tube structures facilitates distinction between the structures, reducing the risk of error and extended surgical time.

In another embodiment of this aspect of the present invention, the lumen walls are pretreated with- an agent to reduce the risk of occlusion of the graft. For example, glow discharge coatings and plasma polymerization methods such as are taught by U.S. Patent No. 4,632,842 to Karwoski et al., impart a property to the flow surface that will reduce or prevent smooth muscle cell build-up within the lumen. The walls can also be pre-coated, for example, with a gelatin or a "time-release" agent that releases small, localized amounts of anticoagulant to the graft site.

The present tubular devices can be manufactured from various biocompatible material. For example, Teflon® brand polytetrafluoroethylene (PTFE), in both its expanded and unexpanded forms, is suitable for use with the invention. Polymer alloys are suitable as well. Dacron® brand polyester fiber, mandrel spun polyurethane, and silicone elastomer fibers are also well suited for use with the invention. Moreover, copolymeric materials such as described in U.S. Patent Nos. 4,187,390 and 4,973,609 can be utilized. These are materials made up of more than one type of monomer and have advantages, as described in the cited patents, in some applications. In a preferred embodiment, the tube structures are manufactured from (PTFE) which has been stretched and/or expanded as described in detail above.

Referring now to the Figures, one embodiment of the device is shown in FIGURE 22, in which the tube structures 410 is joined at one end by a terminal cap assembly 414 with fluid flow through each lumen 412. The tube structures 410 can include a notch near the fused, terminal end of the structures 410 to direct fluid flow through each lumen. The terminal cap assembly 414 is made of any suitable biocompatible material. The terminal cap assembly 414 can optionally be removable to facilitate certain procedures, e.g., thrombectomy. In that example, an incision is made away from the anastoma and needle access area, thereby allowing the clot to be removed without disrupting the intact graft and fluid flow through the lumena.

In the embodiment shown in FIGURE 22A, the tube structures 410 share a divisible wall structure 416 which has a thickness sufficient to allow a user of the apparatus 100 to tear one tube structure 410 apart from another 410' without affecting the integrity of either tube structure. The tube structures can optionally include a built-in stop-gap 418, which prevents a user from dividing the tube structures beyond the point of structural integrity.

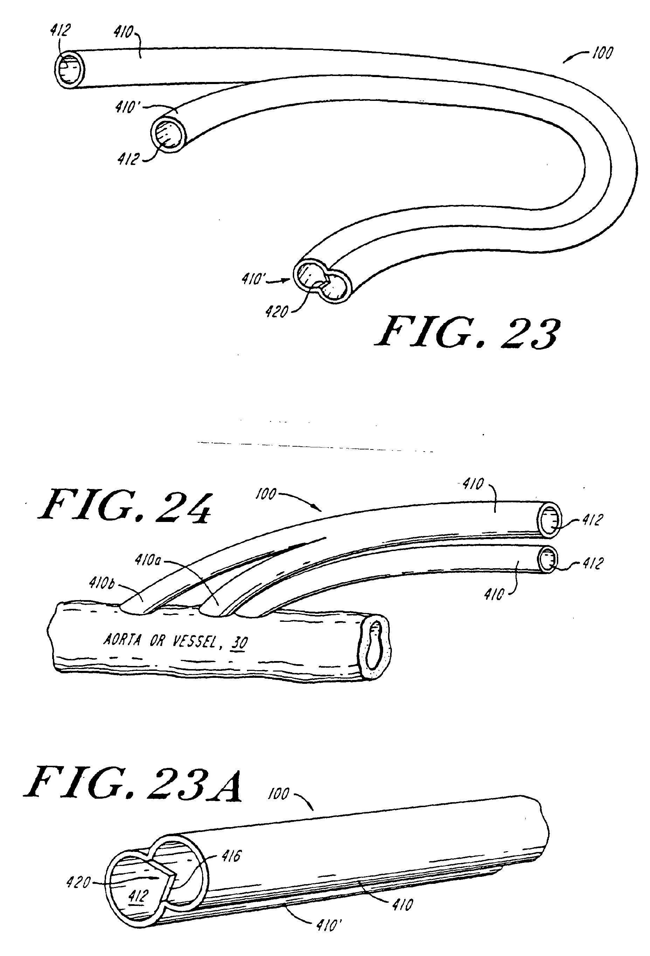

The device 410 can be straight, and relatively rigid, as shown, by example, in FIGURE 22, or can be flexible for configuration in a variety of functional modes, as shown, by example, in FIGURE 23. In that embodiment, the device 100 includes two separable tube structures 410, 410' of relatively flexible structure which can be used for multiple arteriovenous applications. In the embodiment shown in FIGURE 23, the two lumena 412 are of the same diameter for use as a multiple vein return, used, for example, to relieve arterial steal syndrome.

As illustrated in FIGURE 23A, the device can include a divider notch 420 to direct blood flow through the lumena 412 and 412'. The notches can have various configurations, including a V-shaped notch, a rounded notch, or other configurations as may be desirable based on the specific location of the prosthesis and the type of fluid flow desired by the user.

Another embodiment of the present invention is illustrated in FIGURE 24. In that embodiment, the device 100 includes pre-divided tube structures 410a and 410b at the proximal end, instead of a terminal end cap. This embodiment of the invention can be used to graft two or more vessels 30 to feed into one or more lumena.

The extrusion can be performed using dies of predetermined shape using principles known in the art. FIGURES 2 , and 2A and 2B show three orthogonal cross-sectional views of an exemplary die which can be used in the manufacture of a device of the present invention.

Generally, and as illustrated, the die 50 consists of a peripheral support structure 56 encasing a first solid die-piece 52 for forming a first lumen, and second solid die-piece 54 proximal to the first die-piece 52 for forming a secondary lumen. The specific spacing of the first die-piece 52 from the second die-piece 54 depends upon the specific desired prosthesis configuration. As best shown in the cross-section in FIGURE 2A, the die 50 may include an external port 60 for introduction of PTFE paste, or the like, for extrusion. FIGURE 2B shows in cross-section the exemplary die 50 of FIGURE 2, showing aperture 58 for forming membrane (418 of FIGURE 22) of the invention. The manufacture of such dies is understood to be well known in the art.

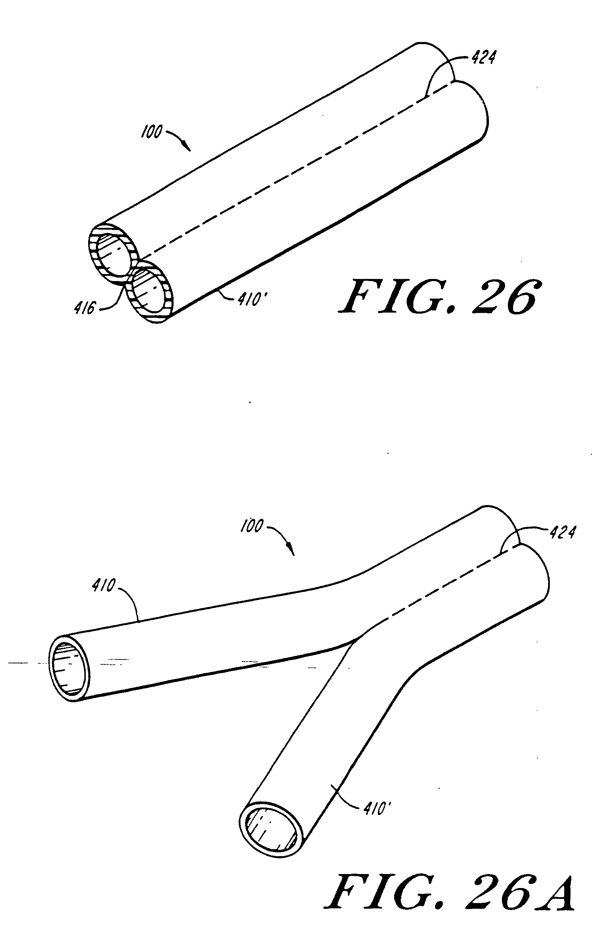

Another embodiment of the invention is illustrated in FIGURES 26 and 26A. FIGURE 26 shows a dilumenal structure 100 having tube structures 410 and 410' joined by a divisible wall 416 which has perforations 424 therein to facilitate separation of the tubes 410 and 410'. FIGURE 26A shows the tubes 410 and 410' partially separated.

In operation, the surgeon surgically exposes the desired region for introduction of the vascular prosthesis of the invention. The desired site may be an area of occlusion or weakness in the patient's arteriovascular system, for example. An interruption of the patient's blood flow is performed, and the surgeon manually separates the tube structures from each other, tearing along the common wall to the desired stop point. Thus, a single divided vascular prosthesis is surgically implanted and sutured or otherwise secured in the desired location. Proper positioning of the prosthesis requires alignment of the lumen with the appropriate blood flow pathway such that the patient's blood flow is diverted through a lumen of the prosthesis. The same procedure is then repeated for the other lumena of the prosthesis, or the extra lumen can be tied off and remain unused.

It should be understood that while several embodiments of the invention have been described in detail, the invention may be embodied in other specific forms without departing from the spirit or essential characteristics thereof. The present embodiments are therefore to be considered in all respects as illustrative and not restrictive, the scope of the invention being indicated by the appended claims rather than by the foregoing description, and all changes coming within the meaning and range of equivalency of the claims are therefore intended to be embraced therein.

Embodiments of the invention may include the features of the following enumerated paragraphs ("paras").
1. An implantable prosthetic device for sustained release of a bioactive material into a fluid flow pathway of a patient comprising
   a body adapted for attachment to said fluid flow pathway, said body defining
   (a) a primary lumen for accommodating fluid flow therethrough, and
   (b) at least one secondary lumen constituting a chamber at least a portion of which is separated from said primary lumen by a permeable wall permitting a bioactive material disposed in said lumen to permeate through said wall and into said primary lumen.
2. A device as set forth in para 1 wherein said bioactive material is a leachable strand of solid material.
3. The device of para 1 further comprising a means for delivering the bioactive material from an external source into said at least one secondary lumen.
4. The device of para 3 wherein said delivery means comprises a biologically activated pump.
5. The device of para 3 wherein said deliver means comprises a mechanical pump.
6. The device of para 5 wherein at least one secondary lumen is filled with said bioactive material prior to implantation.
7. An implantable prosthetic device for delivering a bioactive material into a blood vessel of a patient, the device comprising
   a tubular body adapted for attachment to a blood vessel of the patient, said body defining
   (a) a primary lumen for accommodating blood flow therethrough, and
   (b) at least one secondary lumen at least a portion of which constitutes a chamber which extends along and is separated from said primary lumen by a microporous wall, said at least one secondary lumen being adapted for containing a bioactive material for permeating through said microporous wall into said primary lumen.
8. A device as set forth in para 1 or 7 wherein said fluid flow pathway is a blood flow pathway.
9. A device as set forth in para 1 or 7 wherein said fluid flow pathway is an organ to organ flow pathway.
10. An implantable prosthetic device for delivering a bioactive material into a blood vessel of a patient, the device comprising
   a tubular body consisting of polytetrafluorethylene and adapted for attachment to a blood vessel of the patient, said body defining
   (a) a primary lumen for accommodating blood flow therethrough, and
   (b) a secondary lumen constituting a chamber at least a portion of which is separated from said primary lumen by a microporous wall, said secondary lumen being adapted for containing a bioactive material for permeating through said microporous wall into said primary lumen.
11. A method for delivering a bioactive material to a fluid flow pathway of a patient, the method comprising the steps of
   providing an implantable prosthetic device adapted for surgical connection to the fluid flow pathway, the device comprising
   a body defining a primary lumen for accommodating fluid flow therethrough, and at least one chamber adjacent said primary lumen, each chamber constituted by a secondary lumen at least a portion of which is separated from said primary lumen by a microporous wall,
   surgically grafting said prosthetic device to the fluid flow pathway in the patient to establish fluid flow through said primary lumen, and
   introducing said bioactive material into at least one of the secondary lumena thereby allowing the material to permeate at a controlled rate through the microporous wall into the fluid flow pathway.
12. A method as set forth in para 11 wherein said step of introducing said bioactive material in to said at least one secondary lumena comprises pre-filling at least one of said at least one secondary lumen with said bioactive material prior to said grafting step.
13. The method of para 11 further comprising the step of coating an exterior surface of said tube with a biocompatible material.
14. The method of para 11 further comprising the step of attaching to at least one of the secondary lumena, means for delivering the bioactive material to said at least one secondary lumen.
15. A method as set forth in para 11 wherein said fluid flow pathway is a blood vessel.
16. A method for delivering a bioactive material to a patient's bloodstream, the method comprising the steps of
   providing an implantable prosthetic device adapted for surgical connection to a blood vessel of the patient, the device comprising
   a tubular body consisting of polytetrafluoroethylene and defining a primary lumen for accommodating blood flow therethrough, and a secondary lumen constituting a chamber at least a portion of which is separated from said primary lumen by a microporous wall,
   surgically grafting said prosthetic device to a blood vessel of the patient to establish blood flow through said primary lumen, and
   introducing said bioactive material into the secondary lumen thereby allowing the material to permeate at a controlled rate through the microporous wall into the fluid flow pathway.
17. The method of para 16 wherein said polytetrafluoroethylene is selected from the group consisting of, expanded polytetrafluoroethylene, stretched polytetrafluoroethylene, and stretched and expanded polytetrafluoroethylene.
18. A method as set forth in para 16 wherein said step of introducing said bioactive material into said at least one secondary lumen comprises pre-filling at least one of the secondary lumena with said bioactive material prior to said grafting step.
19. The method of para 16 further comprising the step of coating an exterior surface of said tubular body with a biocompatible material.
20. The method of para 16 further comprising the step of attaching to at least one of said at least one secondary lumen, means for delivering the bioactive material to said at least one secondary lumen.
21. A self-sealing implantable prosthetic device for connection to a fluid flow pathway of a patient, the device comprising
   a. an implantable body of unitary construction adapted for attachment to said fluid flow pathway, said body defining a primary lumen for accommodating fluid flow therethrough and a secondary lumen sharing a common side wall with said primary lumen each of the lumena being bounded by a single continuous surface of the body; and
   b. a non-biodegradable self-sealing elastomeric material disposed in said secondary lumen.
22. The device of para 21 wherein said primary lumen is longer than said secondary lumen.
23. The device of para 21 wherein said body consists of a material selected from the group consisting of: polyester fibers, polyurethane, polysiloxane polymers, and polytetrafluoroethylene.
24. A self-sealing implantable vascular graft device for connection to a blood vessel of a patient, the graft comprising
   a. an implantable body of unitary construction adapted for attachment to a blood vessel of a patient, said body defining a primary lumen for accommodating blood flow therethrough and a secondary lumen sharing a common side wall with said primary lumen each of the lumena being bounded by a single continuous surface provided by the body, and
   b. a non-biodegradable self-sealing elastomeric material disposed in said secondary lumen.
25. The device of para 24 wherein said primary lumen is longer than said secondary lumen.
26. The device of para 24 wherein said body consists of a material selected from the group consisting of: polyester fibers, polyurethane, polysiloxane polymers, and polytetrafluoroethylene.
27. A method for accessing a patient's vascular system comprising the steps of:
   a. providing a vascular graft formed of an implantable body of unitary construction adapted for attachment to a blood vessel of the patient, the body defining a primary lumen for accommodating fluid flow therethrough and a secondary lumen sharing a common side wall with the primary lumen, each of the lumena being bounded by a single, continuous surface provided by the body, the graft further including a non-biodegradable self-sealing elastomeric material disposed in the secondary lumen;
   b. connecting the vascular graft to a blood vessel of the patient; and
   c. accessing the patient's vascular system by passing a cannula through the said secondary lumen, said common side wall, and into said primary lumen.
28. An implantable prosthetic device for connection to a fluid flow pathway of a patient, the device comprising
   a biocompatible microporous wall defining a lumen extending along a longitudinal axis and adapted for accommodating fluid flow therethrough, and
   plural remotely detectible components formed integrally with said wall and extending parallel to said longitudinal axis, said plural remotely detectible components being arranged for indicating the patency of the lumen.
29. A device as set forth in Para 28 wherein said remotely detectible components are formed of a material comprising a radiopaque polymer.
30. A device as set forth in para 28 wherein said remotely detectible components comprise metallic strips.
31. A device as set forth in para 28 wherein said remotely detectible components are formed of a material which has a density different from that of said tubular body.
32. A device as set forth in para 28 wherein said remotely detectible components are arranged substantially equidistant about the circumference of said lumen.
33. An implantable prosthetic device comprising a biocompatible microporous tubular body defining
   a primary lumen adapted for accommodating fluid flow therethrough, and
   at least two secondary lumena extending longitudinally parallel to the primary lumen and arranged equidistant about said primary lumen, and
   a remotely detectible material disposed in said secondary lumena.
34. A device as set forth in para 33 wherein said remotely detectible material is a radiopaque polymer.
35. A device as set forth in para 33 wherein said remotely detectible material is a metallic strip.
36. A device as set forth in para 33 wherein said remotely detectible material has a density different from that of said tubular body.
37. A method for monitoring the position or patency of a vascular graft in a patient, the method comprising the steps of
   a. providing a- vascular graft comprising a biocompatible microporous tubular body defining a primary lumen and at least two secondary lumena each containing remotely detectible material,
   b. implanting the vascular graft in the patient under conditions sufficient to establish blood flow through the primary lumen, and
   c. monitoring the graft by a remote imaging technique.
38. A method as set forth in para 37 wherein said remote imaging technique is selected from the group consisting of x-ray imaging, ultrasonic imaging and magnetic resonance imaging.
39. The method of para 37 wherein the step of monitoring the vascular graft is carried out substantially simultaneously with the step of implanting the vascular graft.
40. The method of para 37 wherein the step of monitoring the vascular graft is carried out after the step of implanting the vascular graft.
41. An implantable prosthetic device for connection to a fluid flow pathway of a patient, the device comprising an implantable body made of biocompatible material adapted for attachment to said fluid flow pathway, said implantable body defining a multiplicity of capillary lumena extending substantially parallel to a longitudinal axis of the body and adapted for accommodating fluid flow therethrough.
42. A device as set forth in para 41 wherein said lumena are separated by microporous walls.
43. A device as set forth in para 41 wherein said microporous walls are sufficiently permeable to allow translumenal communication of cells and extra-cellular fluid.
44. A device as set forth in para 41 wherein said implantable body is of a first diameter and said lumena are of a second diameter, said first diameter being approximately six times greater than said second diameter.
45. A device as set forth in para 41 wherein all of said multiplicity of capillary lumena are of substantially equal diameter.
46. A device as set forth in para 41 wherein each of said lumena is defined by an interior surface, said interior surfaces being treated with a bioactive material selected from the group consisting of protein, glycoprotein, and growth factors.
47. A device as set forth in para 41 wherein said body has a D-shaped cross-section.
48. A device as set forth in para 41 wherein selected capillary lumena are filled with said bioactive material.
49. An implantable prosthetic device for sustained release of a bioactive material into a patient's blood stream, the device comprising an implantable tubular body adapted for attachment to a blood vessel of the patient, said implantable body defining a multiplicity of capillary lumena extending substantially parallel to a longitudinal axis of the body and adapted for accommodating blood flow therethrough, said lumena being separated by microporous walls sufficiently permeable to allow translumenal permeation of a bioactive material.
50. A device as set forth in para 49 wherein said implantable body is of a first diameter and said lumena are of a second diameter, said first diameter being approximately six times greater than said second diameter.
51. A device as set forth in para 49 wherein all of said multiplicity of capillary lumena are of substantially equal diameter.
52. A device as set forth in para 49 wherein each of said lumena is defined by an interior surface, said interior surfaces being treated with a bioactive material selected from the group consisting of protein, glycoprotein, and growth factors.
53. A device as set forth in para 49 wherein said outer wall is D-shaped.
54. A device as set forth in para 49 wherein selected capillary lumena are filled with said bioactive material.
55. A device as set forth in any of paras 1, 2, 7, 10 or 48 wherein said bioactive material is a therapeutic agent.
56. A device as set forth in any of paras 1, 2, 7, 10 or 48 wherein said bioactive material is a diagnostic agent.
57. A method for providing a bioactive material to a fluid flow pathway of a patient, the method comprising the steps of
   providing an implantable body adapted for attachment to said fluid flow pathway and for accommodating fluid flow therethrough, said implantable body defining a multiplicity of capillary lumena extending substantially parallel to a longitudinal axis of the body, said lumena being separated by walls sufficiently permeable to allow translumenal permeation of a bioactive material,
   introducing said bioactive material to selected capillary lumena, and
   surgically grafting said prosthetic device to the fluid flow pathway in the patient to establish fluid flow through said implantable body.
58. A method as set forth in para 57 wherein said introducing step is carried out by prefilling said selected capillary lumena with the bioactive material.
59. A method as set forth in para 57 wherein said introducing step is carried out by coating the interior surfaces of said selected capillary lumena with the bioactive material.
60. A method as set forth in para 57 wherein said implanting step is carried out by placing implantable body in fluid communication with the patient's vascular system.
61. A method as set forth in para 57 wherein said implanting step is carried out by placing the implantable body in fluid communication with an internal organ of the patient.
62. A method as set forth in para 57 further comprising the step of
   seeding said selected capillary lumena prior to said introducing step with material to promote both naturally occurring and genetically dêrived and cultured cell attachment and growth.
63. A method for providing a bioactive material to a patient's blood stream, the method comprising the steps of
   providing an implantable tubular body consisting of polytetrafluoroethylene and adapted for attachment to a blood vessel of the patient, said implantable body defining a multiplicity of capillary lumena extending substantially parallel to a longitudinal axis and adapted for accommodating blood flow therethrough, said lumena being separated by walls sufficiently permeable to allow translumenal permeation of a bioactive material,
   introducing said bioactive material to selected capillary lumena, and
   surgically grafting said prosthetic device to the blood vessel to establish blood flow through said capillary lumena.
64. A method as set forth in para 63 wherein said introducing step is carried out by prefilling said selected capillary lumena with the bioactive material.
65. A method as set forth in para 63 wherein said introducing step is carried out by coating the interior surfaces of said capillary lumena with the bioactive material.
66. A method as set forth in para 63 wherein said implanting step is carried out by placing implantable body in fluid communication with the patient's vascular system.
67. A vascular prosthetic device comprising plural longitudinally parallel tube structures, each of said tube structures comprising a wall defining a longitudinally extending biocompatible exterior surface and an interior lumen of predetermined diameter for channeling fluid flow therethrough, said parallel tube structures being releasably connected to one another via a thickened wall portion extending over at least a portion of the longitudinal extent of said exterior surfaces to permit manual spatial separation of said tube structures.
68. The device of para 67 wherein said tube structures include identifying indicia for distinguishing each of said structures from the other of said structures.
69. An implantable vascular prosthetic device comprising first and second longitudinally parallel tube structures, each of said structures comprising a wall consisting of polytetrafluoroethylene and defining a longitudinally extending biocompatible exterior surface and an interior lumen of predetermined diameter for channeling blood flow therethrough, said lumena being of unequal diameters and converging to form a single lumen at one end of the vascular prosthesis, said tube structures being releasably connected to one another over at least a portion of the longitudinal extent of said exterior surfaces to permit manual spatial separation of said tube structures.
70. The device of para 69 wherein said tube structures are attached to one another via a thickened wall portion.
71. The device of para 69 wherein said tube structures are attached to one another via a bead of material.
72. The device of para 69 wherein said tube structures include identifying indicia for distinguishing each of said structures from the other of said structures.
73. An implantable vascular prosthetic device comprising plural longitudinally parallel tube structures, each of said tube structures comprising a wall defining a longitudinally extending biocompatible exterior surface and an interior lumen of predetermined diameter for channeling fluid flow therethrough, said parallel tube structures being releasably connected to one another via a bead of material extending over at least a portion of the longitudinal extent of said exterior surfaces to permit manual spatial separation of said tube structures.
74. The device of para 73 wherein said tube structures include identifying indicia for distinguishing each of said structures from the other of said structures.
75. An implantable vascular prosthetic device comprising plural longitudinally parallel tube structures, each of said tube structures comprising a wall defining a longitudinally extending biocompatible exterior surface and an interior lumen of predetermined diameter for channeling fluid flow therethrough, said parallel tube structures being releasably connected to one another over at least a portion of the longitudinal extent of said exterior surfaces to permit manual spatial separation of said tube structures wherein the diameter of the lumen of one of the plural tube structures is different from the diameter of the lumen of at least one other of the plural tube structures.
76. An implantable vascular prosthetic device comprising plural longitudinally parallel tube structures, each of said tube structures comprising a wall defining a longitudinally extending biocompatible exterior surface and an interior lumen of predetermined diameter for channeling fluid flow therethrough, said parallel tube structures being releasably connected to one another over at least a portion of the longitudinal extent of said exterior surfaces to permit manual spatial separation of said tube structures wherein at least two of the lumen'a converge to form a single lumen at one end of said tube structure.
77. The device of any of paras 1, 2, 7, 28, 33, 41, 49, 67, 73, 75 or 76 consisting substantially of polytetrafluoroethylene.
78. The device of para 77 wherein said polytetrafluoroethylene is selected from the group consisting of expanded polytetrafluoroethylene, stretched polytetrafluoroethylene, and stretched and expanded polytetrafluoroethylene.
79. The device of any of paras 1, 2, 7, 28, 33, 49, 67, 73, 75 or 76 consisting substantially of a copolymeric material.
80. The device of para 76 wherein said tube structures include identifying indicia for distinguishing each of said structures from the other of said structures.

## Claims

1. An implantable prosthetic device for connection to a fluid flow pathway of a patient, the device comprising an implantable body made of a single biocompatible material and extruded as a single body with microporous interior walls, said extruded single body having a shape to attach to said fluid flow pathway, said single body further having a multiplicity of capillary lumina (314) therein defined by said microporous interior walls (315) and extending substantially parallel to each other along a longitudinal axis of the body for accommodating fluid flow along said axis when attached to said fluid flow pathway.

2. A device as set forth in claim 1 wherein said microporous walls (315) are sufficiently permeable to allow transluminal communication of cells and extra-cellular fluid.

3. A device as set forth in claim 1 wherein said implantable body is of a first diameter and said lumina are of a second diameter, said first diameter being approximately six times-greater-than said second diameter.

4. A device as set forth in claim 1 wherein all of said multiplicity of capillary lumina (314) are of substantially equal diameter.

5. A device as set forth in claim 1 wherein each of said lumina is defined by an interior surface, said interior surfaces being treated with a bioactive material selected from the group consisting of protein, glycoprotein, and growth factors.

6. A device as set forth in claim 1 wherein said body has a D-shaped cross-section.

7. A device as set forth in claim 1 wherein said implantable body consists of polytetrafluoroethylene.

8. A device as set forth in claim 7 wherein said polytetrafluoroethylene is selected from the group consisting of expanded polytetrafluoroethylene, stretched polytetrafluoroethylene, and expanded and stretched polytetrafluoroethylene.

9. A device as set forth in claim 1 wherein said implantable body consists of a material including copolymers.

10. A device as set forth in claim 1 wherein selected capillary lumina (314) are filled with a bioactive material.

11. A device as set forth in claim 10 wherein said bioactive material is a therapeutic agent.

12. A device as set forth in claim 10 wherein said bioactive material is a diagnostic agent.
